(19) Europäisches Patentamt
European Patent Office
Office européen des brevets

(11) **EP 1 398 381 A1**

(12) **EUROPEAN PATENT APPLICATION**

(43) Date of publication:
**17.03.2004 Bulletin 2004/12**

(21) Application number: **02447168.2**

(22) Date of filing: **06.09.2002**

(51) Int Cl.7: **C12N 15/53**, C12N 9/02, C11D 3/386, C12N 1/21, C12N 5/10

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(71) Applicant: **THE PROCTER & GAMBLE COMPANY**
**Cincinnati, Ohio 45202 (US)**

(72) Inventors:
- **Zorn, Holger**
  **30926 Seelze (DE)**
- **Langhoff, Sabine**
  **30451 Hannover (DE)**
- **Berger, Ralf G.**
  **30455 Hannover (DE)**

(74) Representative: **Engisch, Gautier et al**
**B.V. Procter & Gamble Services Company SA,**
**100 Temselaan**
**1853 Strombeek-Bever (BE)**

Remarks:
The sequence listing, which is published as annex to the application documents, was filed after the date of filing. The applicant has declared that it does not include matter which goes beyond the content of the application as filed.

(54) **A microbial oxidoreductase**

(57) A microbial oxidoreductase capable of converting carotenoid substrates is provided. The oxidoreductase may be obtained from basidiomycetes, especially *Lepista irina.* An isolated nucleic acid encoding an open reading frame for the oxidoreductase is also provided, together with a polypeptide encoded by the nucleic acid. Carotenoids may be converted by incubation with the enzyme. Applications for the oxidoreductase include inclusion in detergent compositions for use in the treatment of stains, especially carotene-based stains.

**Figure 1**

cDNA sequence

GGATCCGGCCATTATGGCCGGGGAAGATCCCCAGTCTTACAACCCACTGC
A**ATG**TCTTTCAAGACGCTCTCCGCTCTCGCGCTTGCGCTCGGCGCCGCCG
TCCAGTTCGCGAGTGCTGCTGTGCCTCTCGTCCAGAAACGCGCAACTTGC
GCCGACGGACGCACCACCGCAAATGCTGCATGTTGCGTTCTGTTCCCCAT
CCTCGATGACATCCAAGAAAACCTCTTCGACGGTGCCCAGTGTGGAGAAG
AGGTACACGAGTCCCTTCGTTTGACTTTCCACGATGCAATCGGTTTCTCTC
CTACTTTAGGCGGAGGAGGAGCTGACGGTTCCATCATCGCGTTCGACACC
ATTGAGACTAATTTCCCCGCCAATGCTGGCATCGATGAAATCGTCAGCGCT
CAGAAGCCATTCGTGGCTAAACACAACATCTCCGCCGGCGACTTCATTCAA
TTTGCTGGCGCCGTTGGAGTCTCCAACTGCCCTGGTGGTGTCAGGATTCC
TTTCTTCTTGGGTCGCCCGGATGCCGTGGCGGCCTCCCCGGACCACCTC
GTGCCAGAGCCTTTTGATTCTGTTGACACCATTCTTGCCAGAATGGGTGAC
GCAGGCTTCAGTCCCGTCGAGGTTGTTTGGCTCCTGGCTTCGCACTCCAT
TGCCGCTGCCGACAAGGTTGACCCATCGATTCCTGGAACGCCATTCGATT
CAACCCCCGGAGTTTTTGATTCTCAATTCTTCATCGAAACGCAACTTAAAG
GCAAACTCTTCCCAGGCACTGCTGACAACAAGGGAGAAGCCCAATCTCCA
TTGCAAGGAGAGATCAGGCTTCAGTCCGATCACTTGTTGGCTAGAGACCC
CCAGACTGCCTGTGAATGGCAGTCCATGGTTAACAACCAACCGAAGATTC
AGAACCGTTTCGCTGCTACCATGTCGAAGATGGCTCTTCTTGGCCAAGACA
AGACCAAATTGATTGACTGTTCTGATGTTATCCCCACCCCTCCTGCCCTTG
TCGGAGCGGCCCACTTGCCGGCGGGATTTTCTCTTAGCGATGTAGAGCAA
GCGTGCGCCGAGACCCCTTTCCCTGCTCTTACTGCTGACCCAGGCCCAGT
AACCTCTGTCCCTCCCGTCCCT**GGATCGTAA**ATGCTTCGATACCTGAATAT
GCTCGTTCTGCTGCGCTGAATTTCCAACTTTTGCCATTGGGTCTGTATTCG
ATTCTAGATGTTTGTGATATCAACTGTGTATAAATGATCTTTTGAAATATACT
TTTTTCTGCGGAGPolyA

EP 1 398 381 A1

SEQUENCE LISTING

<110> The Procter & Gamble Company

<120> A Microbial Oxidoreductase

<130> EP02447168.2

<140> EP02447168.2
<141> 2002-09-06

<160> 2

<170> PatentIn version 3.1

<210> 1
<211> 1274
<212> DNA
<213> Lepista irina

<400> 1
ggatccggcc attatggccg gggaagatcc ccagtcttac aacccactgc aatgtctt
tc 60

aagacgctct ccgctctcgc gcttgcgctc ggcgccgccg tccagttcgc gagtgctg
ct 120

gtgcctctcg tccagaaacg cgcaacttgc gccgacggac gcaccaccgc aaatgctg
ca 180

tgttgcgttc tgttccccat cctcgatgac atccaagaaa acctcttcga cggtgccc
ag 240

tgtggagaag aggtacacga gtcccttcgt ttgactttcc acgatgcaat cggtttct
ct 300

cctactttag gcggaggagg agctgacggt tccatcatcg cgttcgacac cattgaga
ct 360

aatttccccg ccaatgctgg catcgatgaa atcgtcagcg ctcagaagcc attcgtgg
ct 420

aaacacaaca tctccgccgg cgacttcatt caatttgctg gcgccgttgg agtctcca
ac 480

tgccctggtg gtgtcaggat tcctttcttc ttgggtcgcc cggatgccgt ggcggcct
cc 540

ccggaccacc tcgtgccaga gccttttgat tctgttgaca ccattcttgc cagaatgg
gt 600

gacgcaggct tcagtcccgt cgaggttgtt tggctcctgg cttcgcactc cattgccg

ct 660

gccgacaagg ttgacccatc gattcctgga acgccattcg attcaacccc cggagttt
tt 720

gattctcaat tcttcatcga aacgcaactt aaaggcaaac tcttcccagg cactgctg
ac 780

aacaagggag aagcccaatc tccattgcaa ggagagatca ggcttcagtc cgatcact
tg 840

ttggctagag acccccagac tgcctgtgaa tggcagtcca tggttaacaa ccaaccga
ag 900

attcagaacc gtttcgctgc taccatgtcg aagatggctc ttcttggcca agacaaga
cc 960

aaattgattg actgttctga tgttatcccc acccctcctg cccttgtcgg agcggccc
ac 1020

ttgccggcgg gattttctct tagcgatgta gagcaagcgt gcgccgagac ccctttcc
ct 1080

gctcttactg ctgacccagg cccagtaacc tctgtccctc ccgtccctgg atcgtaaa
tg 1140

cttcgatacc tgaatatgct cgttctgctg cgctgaattt ccaacttttg ccattggg
tc 1200

tgtattcgat tctagatgtt tgtgatatca actgtgtata aatgatcttt tgaaatat
ac 1260

tttttctgc ggag
1274

<210> 2
<211> 360
<212> PRT
<213> Lepista irina

<400> 2

Met Ser Phe Lys Thr Leu Ser Ala Leu Ala Leu Ala Leu Gly Ala Ala
1 5 10 15

Val Gln Phe Ala Ser Ala Ala Val Pro Leu Val Gln Lys Arg Ala Thr
20 25 30

Cys Ala Asp Gly Arg Thr Thr Ala Asn Ala Ala Cys Cys Val Leu Phe
35 40 45

Pro Ile Leu Asp Asp Ile Gln Glu Asn Leu Phe Asp Gly Ala Gln Cys
50 55 60

Gly Glu Glu Val His Glu Ser Leu Arg Leu Thr Phe His Asp Ala Ile
65 70 75 80

Gly Phe Ser Pro Thr Leu Gly Gly Gly Gly Ala Asp Gly Ser Ile Ile
85 90 95

Ala Phe Asp Thr Ile Glu Thr Asn Phe Pro Ala Asn Ala Gly Ile Asp
100 105 110

Glu Ile Val Ser Ala Gln Lys Pro Phe Val Ala Lys His Asn Ile Ser
115 120 125

Ala Gly Asp Phe Ile Gln Phe Ala Gly Ala Val Gly Val Ser Asn Cys
130 135 140

Pro Gly Gly Val Arg Ile Pro Phe Phe Leu Gly Arg Pro Asp Ala Val
145 150 155 160

Ala Ala Ser Pro Asp His Leu Val Pro Glu Pro Phe Asp Ser Val Asp

165 170 175

Thr Ile Leu Ala Arg Met Gly Asp Ala Gly Phe Ser Pro Val Glu Val
180 185 190

Val Trp Leu Leu Ala Ser His Ser Ile Ala Ala Ala Asp Lys Val Asp
195 200 205

Pro Ser Ile Pro Gly Thr Pro Phe Asp Ser Thr Pro Gly Val Phe Asp
210 215 220

Ser Gln Phe Phe Ile Glu Thr Gln Leu Lys Gly Lys Leu Phe Pro Gly
225 230 235 240

Thr Ala Asp Asn Lys Gly Glu Ala Gln Ser Pro Leu Gln Gly Glu Ile
245 250 255

Arg Leu Gln Ser Asp His Leu Leu Ala Arg Asp Pro Gln Thr Ala Cys
260 265 270

Glu Trp Gln Ser Met Val Asn Asn Gln Pro Lys Ile Gln Asn Arg Phe
275 280 285

Ala Ala Thr Met Ser Lys Met Ala Leu Leu Gly Gln Asp Lys Thr Lys
290 295 300

```
Leu Ile Asp Cys Ser Asp Val Ile Pro Thr Pro Pro Ala Leu Val Gly
305                 310                 315                 320

Ala Ala His Leu Pro Ala Gly Phe Ser Leu Ser Asp Val Glu Gln Ala
                325                 330                 335

Cys Ala Glu Thr Pro Phe Pro Ala Leu Thr Ala Asp Pro Gly Pro Val
            340                 345                 350

Thr Ser Val Pro Pro Val Pro Gly
        355                 360
```

## Description

This invention relates to a microbial oxidoreductase capable of converting carotenoid substrates, detergent compositions comprising the oxidoreductase and methods for converting carotenoids for the production of carotenoid-derived products for use as food, cosmetics and perfume ingredients.

Carotene and other carotenoid compounds are very common ingredients in a wide range of food and cosmetic products.

Due to their colour, carotenoid substances often present problems in the form of stains on fabrics, which are difficult to remove using conventional detergents. So -called "difficult" stains include those based on tomato and pasta sauce, carrot based juices and baby food, green coloured vegetables and grass.

Conventional detergents employed to remove such difficult stains and using enzyme systems often cause fading of sensitive dyes. Such systems are typically based on laccase or peroxide enzymes.

There is a need for enzymes capable of efficiently cleaving carotenoid substrates, to provide enzymatic methods for converting carotenoid substances and for treating carotenoid-based stains.

Carotenoids are present in many natural products such as fruit and vegetables. Examples include carrots, spinach and marigold. It is known that flavourings and fragrancing compounds for use in foods, cosmetics, perfumes and the like can be prepared from such carotenoids.

The most important carotenoids which can be cleaved to produce flavourings and fragrancing compounds are any substances with a carotene backbone, in particular with a β-carotene or capsanthin backbone, more particularly α- and β-carotene, lutein, lycopene, antheraxanthin, capsanthin, zeaxanthin, violaxanthin, astaxanthin, canthaxanthin, luteoxanthin, neoxanthin, and the respective apo-carotenoids.

The products of carotenoid cleavage play a major role in the detergent, food and perfume industry. Among the cleavage products, the so-called nor-isoprenoids, in particular the C-13-cleavage products, are of particular interest. These compounds are also very common in nature and are derived from carotenoids in naturally occurring processes. This is demonstrated by the fact that during the ripening process of fruits, the concentration of carotenoids decreases proportionately to the increase in the concentration of nor-isoprenoids.

Carotenoids can be cleaved in various ways to give rise to different nor-isoprenoids, e.g. to C-9 isophorone, C-10 safranal, C-13 ionone or C-15 abscisinic acid, depending on which double bond is cleaved. Ionones are the primary cleavage products of carotenoids. The most important secondary cleavage products are damascenones and damascones, as well as theaspiranes, vitispiranes and edulanes. The primary and secondary cleavage products are naturally occurring compounds. For example, α- and β-ionone, di-hydro-actinidiolide, theaspirone and 2,2,6-tri-methyl-clyclohexanone derivatives occur in herbal infusions.

The conventional method of producing carotenoid-derived products is by co-oxidation. The reaction is catalysed by lipoxygenases. However, this process is not very efficient. The positions of the double bonds of the carotenoid precursor are retained in the series of conversion products. For example, delta-damascone is generated by an enzymatic isomerisation reaction. The generation of its immediate precursors, α- or β-damascone, can be carried out using a series of oxygen-introducing enzymes such as oxidases, oxygenases or peroxidases.

WO 94/08028 describes a method for enzymatic preparation of aroma chemicals, particularly ionones and $C_6$ to $C_{10}$ aldehydes, using a lipoxygenase (co-oxidation). This method yields mainly α-ionone, whereas only trace amounts of β-ionone are produced.

The desired aroma chemicals can also be synthesised chemically. The chemosynthesis of some perfume ingredients, for instance macrocyclic musk such as muscenone, is associated with some disadvantages, in particular high dilutions and expensive reagents.

A carotenoid cleaving enzyme present in herbivores, some carnivores and birds was discovered in the 1950s. However, there is no knowledge of any microbial enzymes capable of efficiently cleaving carotenoids.

Accordingly, there is also a need for an improved method of preparing carotenoid-derived products of use as flavourings and aroma chemicals.

It has unexpectedly been found that an enzyme isolated from *Lepista irina,* a basidiomycete, exhibits a very high conversion of carotenoid substrates, in particular β,β-carotene and its derivatives. Other carotenoid substrates capable of being converted by the enzyme include α-carotene (β,ε-carotene) and derivatives. β-ionone is the main cleavage product of β,β-carotene.

In a first aspect, the present invention provides an isolated nucleic acid encoding an open reading frame for a carotene-degrading oxidoreductase, comprising

(a) a sequence according to SEQ ID NO: 1, or
(b) a sequence having 75%, preferably 80%, more preferably 90%, more preferably 95%, or more sequence identity with the sequence according to (a), or
(c) a sequence capable of hybridising to the sequence of (a) and/or (b) under stringent conditions, and/or

(d) a sequence that is complementary to (a), (b) and/or (c).

SEQ ID NO: 1 is set out in Figure 1 hereto and in the sequence listing. The sequence shown in SEQ ID NO: 1 is derived from *Lepista irina. Lepista irina* is a commercially available organism (CBS 458.79, Centraalbureau voor Schimmelcultures, Baarn, NL).

The nucleic acid according to SEQ ID NO: 1 comprises an open frame (ORF) coding for 361 amino acids. SEQ ID NO: 2, set out in Figure 2 hereto, shows the corresponding protein sequence. The enzyme encoded is a polyvalent peroxidase which, for the purposes of this specification, will be referred to as oxidoreductase.

For the purposes of the present invention, "hybridising under stringent conditions" is preferably defined as set out in Sambrook et al "Molecular Cloning: A Laboratory Manual", Cold Spring Harbor Laboratory Press (1989), Chapter 1.101-1.104. Preferably, a stringent hybridisation means that after a 1 hour wash with 1 x SSC and 0.1% SDS at 50°C, preferably 55°C, more preferably 62°C, most preferably 68°C; in particular for 1 hour with 0.2 x SSC and 0.1% SDS at 50°C, preferably 55°C, more preferably 62°C, most preferably 68°C, a positive hybridisation signal is still detectable. A nucleic acid sequence which hybridises under those conditions with the nucleic acid sequence according to SEQ ID NO: 1 is a nucleic acid of the present invention.

The nucleic acid of the present invention is preferably a DNA. However, it can also be an RNA or comprise nucleic acid analogues. Preferably, the nucleic acid comprises a sequence having 75%, preferably 80%, more preferably 90%, more preferably 95%, or more sequence identity with the sequence according to SEQ ID NO:1 or sequences hybridising therewith.

The present invention also provides a vector comprising the sequence of the nucleic acid as shown in SEQ ID NO:1 as well as a cell transformed with such a nucleic acid or with such a vector. The vector backbone suitable for inserting the nucleic acid sequence of the present invention can be chosen by the skilled person. The vector can be a suitable eukaryotic or prokaryotic vector and preferably comprises all the necessary control sequences, such as promoters and enhancers.

In addition, the invention provides a cell transformed with a nucleic acid sequence or vector of the present invention. Another related aspect provides a cell culture comprising the transformed cells of the invention in a suitable cell culture medium. Suitable cells to be transformed with the nucleic acid or vector of the present invention are microbial cells, preferably bacterial or fungal cells.

A related aspect of the invention is a polypeptide encoded by the nucleic acid of the invention according to SEQ ID NO: 1.

The polypeptide is preferably a polypeptide having,

(a) an amino acid sequence according to SEQ ID NO: 2,
(b) an amino acid sequence with 70%, preferably 80%, more preferably 90% homology with (a), and/or
(c) an amino acid sequence which is immunologically cross-reactive with (a) and/or (b).

SEQ ID NO: 2 is set out in Figure 2 and in the sequence listing.

The polypeptide of the present invention represents an oxidoreductase capable of converting carotenoid substrates.

Carotenoid substrates that can be converted using the polypeptide of the present invention are compounds having the basic structure and carbon skeleton depicted in the general formulae I, II and III.

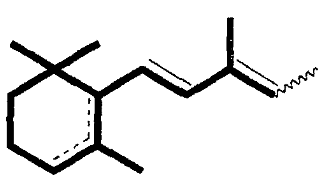

Formula I

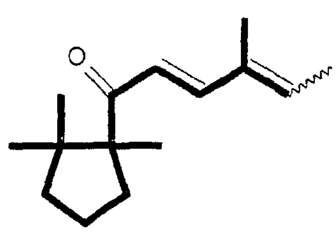

Formula II

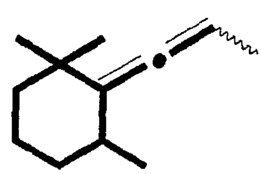

Formula III

References in this specification to carotenoid substrates are to include compounds having the general structure of one of formulae I, II or III, together with all hydroxylated and oxo-functionalised derivatives thereof as well as all naturally occurring stereoisomers.

Preferred substrates include α- and β-carotenoid substrates, in particular β, β-carotene, α-carotene, lycopene, capsanthin, lutein, antheraxanthin, violaxanthin, zeaxanthin, astaxanthin, canthaxanthin, luteoxanthin, neoxanthin and the respective apo-carotenoids.

The oxidoreductase of the present invention is characterised by having a molecular weight of about 50 kDa and an isoelectric point of about 3.75 (determined as described in the examples below).

The oxidoreductase is capable of cleaving carotenoids asymmetrically, thus releasing the desired cleavage products, in particular ionones, more preferably α- and β-ionones. These products are very useful as fragrances, flavours, aroma chemicals and food additives, and also for cosmetics, perfumes and similar.

In general, all enzymatic cleavage products obtainable by using the oxidoreductase of the present invention may be used. Preferred carotenoid derived products include in particular ionones, more preferably α- and β-ionones. Specific preferred examples of such cleavage products include β-ionone, dihydroactinidiolide, 2-hydroxy-2,6,6-trimethylcyclohexanone, β-cyclocitral (cleavage products of β-carotene) or grasshopper ketone (cleavage product of neoxanthin).

Heretofore, it has been necessary to include hydrogen peroxide in enzymatic compositions for converting carotenoids. The oxidoreductase has the unexpected characteristic of being able to cleave carotenoids in the absence of peroxides.

Another aspect of the present invention provides detergent compositions comprising a microbial oxidoreductase capable of converting carotenoid substates. The oxidoreductase is preferably the oxidoreductase of this invention.

The enzyme of the invention may be used in the detergent compositions in its wild-type form. Alternatively, it can be genetically engineered to be adapted to certain detergent applications, for example to improve the stability and activity in a broad pH range (6 to 12), in the presence of surfactants and/or chelate-containing aqueous solutions.

The detergent compositions in which the enzyme of the invention may be incorporated comprise a variety of components, and levels of incorporation thereof will depend on the physical form of the composition, and the nature of the cleaning operation for which it is to be used. Indeed, the detergent compositions herein include laundry detergents as well as hard surface cleaners, hand dishwashing or automatic dishwashing detergents. The detergent compositions herein can be liquid, paste, gels, bars, tablets, spray, foam, powder or granular. Granular compositions can also be in "compact" form and the liquid compositions can also be in a "concentrated" form. Tablet compositions can be in single phase or multiple phase form.

When formulated as compositions for use in manual dishwashing methods the compositions herein typically contain a surfactant and preferably other detergent compounds selected from organic polymeric compounds, suds enhancing agents, group II metal ions, solvents, hydrotropes and additional enzymes.

When formulated as compositions suitable for use in a laundry machine washing method, the compositions herein typically contain both a surfactant and a builder compound and additionally one or more detergent components preferably selected from organic polymeric compounds, bleaching agents, additional enzymes, suds suppressors, dispersants, lime-soap dispersants, soil suspension and anti-redeposition agents and corrosion inhibitors. Laundry compositions can also contain softening agents, as additional detergent components.

When formulated as compositions suitable for use in a machine dish wash method, the compositions herein typically contain a low foaming nonionic surfactant, a builder system, and one or more components preferably selected from organic polymeric compounds, bleaching agents, additional enzymes, suds suppressors, dispersants, lime-soap dispersants, soil suspension and anti-redeposition agents and corrosion inhibitors.

The compositions herein can also be used as detergent additive products in solid or liquid form. Such additive products are intended to supplement or boost the performance of conventional detergent compositions and can be added at any stage of the cleaning process.

If needed the density of the laundry detergent compositions herein ranges from 400 to 1200 g/litre, preferably 500 to 950 g/litre of composition measured at 20°C. The "compact" form of the compositions herein is best reflected by density and, in terms of composition, by the amount of inorganic filler salt; inorganic filler salts are conventional ingredients of detergent compositions in powder form; in conventional detergent compositions, the filler salts are present in substantial amounts, typically 17-35% by weight of the total composition. In the compact compositions, the filler salt is present in amounts not exceeding 15% of the total composition, preferably not exceeding 10%, most preferably not exceeding 5% by weight of the composition. The inorganic filler salts, such as meant in the present compositions are selected from the alkali and alkaline-earth-metal salts of sulphates and chlorides. A preferred filler salt is sodium sulphate.

Liquid detergent compositions according to the present invention can also be in a "concentrated form", in such case, the liquid detergent compositions according the present invention will contain a lower amount of water, compared to conventional liquid detergents. Typically the water content of the concentrated liquid detergent is preferably less than 40%, more preferably less than 30%, most preferably less than 20% by weight of the detergent composition.

The compounds which are generally suitable for use in detergents are described as follows.

Detergent compositions typically comprise a surfactant system wherein the surfactant can be selected from cationic, nonionic and/or conventional anionic and/or mixtures thereof. Also suitable are ampholytic and/or zwitterionic and/or semi-polar surfactants. The surfactant system is typically present at a level of from 0.1% to 60% by weight. More preferred levels of incorporation are 1% to 35% by weight, most preferably from 1% to 30% by weight of the detergent compositions.

The detergent composition may comprise one or more bleaching agents. Bleaching agents that can be used encompasses peroxygen bleaches and halogen bleaching agents. Examples of peroxygen bleaches are inorganic perhydrate bleaches, typically percarbonates and perborates, use alone or in combination with bleach activators surch as TAED. Examples of hypohalite bleaching agents, for example, include trichloro isocyanuric acid and the sodium and potassium dichloroisocyanurates and N-chloro and N-bromo alkane sulphonamides. Such materials are normally added at 0.5-10% by weight of the finished product, preferably 1-5% by weight.

Bleaching agents other than oxygen bleaching agents are also known in the art and can be utilized herein. One type of non-oxygen bleaching agent of particular interest includes photoactivated bleaching agents such as the sulfonated zinc and/or aluminum phthalocyanines. These materials can be deposited upon the substrate during the washing process. Upon irradiation with light, in the presence of oxygen, such as by hanging clothes out to dry in the daylight, the sulfonated zinc phthalocyanine is activated and, consequently, the substrate is bleached. Preferred zinc phthalocyanine and a photoactivated bleaching process are described in U.S. Patent 4,033,718. Typically, detergent compositions will contain about 0.025% to about 1.25%, by weight, of sulfonated zinc phthalocyanine.

The detergent compositions herein can further comprise a builder. Any conventional builder system is suitable for use herein including aluminosilicate materials, silicates, polycarboxylates, alkyl- or alkenyl-succinic acid and fatty acids, materials such as ethylenediamine tetraacetate, diethylene triamine pentamethyleneacetate, metal ion sequestrants such as aminopolyphosphonates, particularly ethylenediamine tetramethylene phosphonic acid and diethylene triamine pentamethylenephosphonic acid.

Phosphate builders can also be used herein. Inorganic aluminosilicates, commonly known as zeolites are also suitable for use herein.

Detergency builder salts are normally included in amounts of from 5% to 80% by weight of the composition preferably from 10% to 70% and most usually from 30% to 60% by weight.

The detergent compositions can, in addition to the enzyme herein, further comprise one or more enzymes which provide cleaning performance, fabric care and/or sanitisation benefits. Said enzymes include enzymes selected from cellulases, hemicellulases, peroxidases, proteases, gluco-amylases, amylases, xylanases, lipases, phospholipases, esterases, cutinases, other pectinases, keratanases, reductases, oxidases, phenoloxidases, lipoxygenases, ligninases, pullulanases, tannases, pentosanases, malanases, β-glucanases, arabinosidases, hyaluronidase, chondroitinase, laccase, pectin lyase, pectate lyase or mixtures thereof. A preferred combination is a detergent composition having a cocktail of conventional applicable enzymes like protease, amylase, lipase, cutinase and/or cellulase in conjunction with one or more plant cell wall degrading enzymes.

Enzymes are normally incorporated in the detergent composition at a total level of from 0.0001 % to 2% of pure enzyme by weight of the detergent composition. The enzymes can be added as separate single ingredients (prills, granulates, stabilized liquids, etc. containing one enzyme ) or as mixtures of two or more enzymes (e.g. cogranulates ).

Technologies which provide a type of colour care benefit can also be included. Examples of these technologies are metallo catalysts for colour maintenance. Such metallo catalysts are described in EP-B-0 596 184. Dye fixing agents, polyolefin dispersion for anti-wrinkles and improved water absorbancy, perfume and amino-functional polymer, disclosed in EP-A-0 931 133, for colour care treatment and perfume substantivity are further examples of colour care / fabric care technologies.

Fabric softening agents can also be incorporated into detergent compositions in accordance with the present invention. These agents may be inorganic or organic in type. Inorganic softening agents are exemplified by the smectite clays disclosed in GB-A-1 400 898 and in USP 5,019,292. Organic fabric softening agents include the water insoluble tertiary amines as disclosed in GB-A1 514 276 and EP-B0 011 340 and their combination with mono C12-C14 quaternary ammonium salts are disclosed in EP-B-0 026 527 and EP-B-0 026 528 and di-long-chain amides as disclosed in EP-B-0 242 919. Other useful organic ingredients of fabric softening systems include high molecular weight polyethylene oxide materials as disclosed in EP-A-0 299 575 and 0 313 146.

Levels of smectite clay are normally in the range from 2% to 20%, more preferably from 5% to 15% by weight, with the material being added as a dry mixed component to the remainder of the formulation. Organic fabric softening agents such as the water-insoluble tertiary amines or dilong chain amide materials are incorporated at levels of from 0.5% to 5% by weight, normally from 1% to 3% by weight whilst the high molecular weight polyethylene oxide materials and the water soluble cationic materials are added at levels of from 0.1% to 2%, normally from 0.15% to 1.5% by

weight. These materials are normally added to the spray dried portion of the composition, although in some instances it may be more convenient to add them as a dry mixed particulate, or spray them as molten liquid on to other solid components of the composition.

The detergent compositions herein may also optionally contain one or more iron and/or manganese chelating agents. Such chelating agents can be selected from the group consisting of amino carboxylates, amino phosphonates, polyfunctionally-substituted aromatic chelating agents and mixtures therein, all as hereinafter defined. Without intending to be bound by theory, it is believed that the benefit of these materials is due in part to their exceptional ability to remove iron and manganese ions from washing solutions by formation of soluble chelates.

If utilized, these chelating agents will generally comprise from about 0.1% to about 15% by weight of the detergent compositions herein. More preferably, if utilized, the chelating agents will comprise from about 0.1% to about 3.0% by weight of such compositions.

Other components used in detergent compositions may be employed, such as soil-suspending agents, soil-release agents, optical brighteners, abrasives, bactericides, tarnish inhibitors, suds suppressors, dye transfer inhibitors, coloring agents, and/or encapsulated or non-encapsulated perfumes, dispersants.

The compositions of the invention may be used in essentially any washing or cleaning methods, including soaking methods, pretreatment methods and methods with rinsing steps for which a separate rinse aid composition may be added.

The process described herein comprises contacting fabrics, dishware or any other hard surface with a cleaning solution in the usual manner and exemplified hereunder. A conventional laundry method comprises treating soiled fabric with an aqueous liquid having dissolved or dispensed therein an effective amount of the laundry detergent and/or fabric care composition. A preferred machine dishwashing method comprises treating soiled articles with an aqueous liquid having dissolved or dispensed therein an effective amount of the machine diswashing or rinsing composition. A conventional effective amount of the machine dishwashing composition means from 8-60 g of product dissolved or dispersed in a wash volume from 3-10 litres. According to a manual dishwashing method, soiled dishes are contacted with an effective amount of the diswashing composition, typically from 0.5-20g (per 25 dishes being treated). Preferred manual dishwashing methods include the application of a concentrated solution to the surfaces of the dishes or the soaking in large volume of dilute solution of the detergent composition. A conventional hard surface method comprises treating soiled hard items/surfaces with e.g. a sponge, brush, clothe, etc. with an aqueous liquid having dissolved or dispensed therein an effective amount of the hard surface cleaner and/or with such composition undiluted. It also encompasses the soaking of a hard item in a concentrated solution or in a large volume of dilute solution of the detergent composition.

The process of the invention is conveniently carried out in the course of the cleaning process. The method of cleaning is preferably carried out at 5°C to 95°C, especially between 10°C and 60°C. The pH of the treatment solution is preferably from 7 to 12.

The oxidoreductase of the present invention is especially suited for incorporation in neutral pH heavy-duty liquid detergents (HDL) and in non-bleach cleaning products. One advantage of the present invention is that the oxidoreductase is active in the absence of peroxides, in particular hydrogen peroxide. It is therefore possible to prepare detergent compositions according to the present invention which are substantially free of bleaching agents, in particular hydrogen peroxide.

Further, the present invention provides a method for treating carotene-comprising stains, comprising contacting the material bearing the stain with the oxidoreductase of the present invention or a detergent composition of the present invention. Preferably, the stained material and the oxidoreductase are contacted for a time period sufficient to substantially remove the stain.

This method is preferably carried out in the absence of bleaching agents, in particular hydrogen peroxide. However, it is possible to use a detergent as described above and use the enzyme of the invention in combination with conventional detergent systems and/or enzyme systems.

Another aspect of the present invention provides a method for producing carotene derived products from a carotenoid substrate, comprising:

(a) contacting the carotenoid substrate with the oxidoreductase of the present invention, and
(b) incubating the mixture of carotenoid substrate and oxidoreductase.

The incubation is preferably carried out for a time period sufficient for the oxidoreductase to asymmetrically cleave the substrate. The carotenoid substrate is preferably emulsified with a suitable surfactant, such as a polyoxyethylene sorbitan ester (commercially available as the Tween™ range of products) and incubated either with the isolated enzyme or with enzyme containing culture supernatant in a buffered solution at a suitable pH, preferably in the range of from 3 to 5, for sufficient time, typically from 30 min to 3 h. The cleavage products may be recovered by techniques known in the art, such as solvent extraction or adsorption.

The oxidoreductase of the present invention is active in converting a wide range of carotenoid substrates, in particular β,β-carotene, α-carotene, lycopene, capsanthin, lutein, antheraxanthin, violaxanthin, zeaxanthin, astaxanthin, canthaxanthin, luteoxanthin, neoxanthin and the respective apo-carotenoids. The enzyme is particularly active in the conversion of α-carotene, β,β-carotene, capsanthin, lycopene, antheraxanthin, violaxanthin and neoxanthin.

Preferred carotenoid derived products include in particular ionones, more preferably α- and β-ionones.

The carotenoid derived products obtained by this method are useful as fragrances and/or flavours in perfumes, cosmetics and/or foods.

By using the oxidoreductase of the present invention, it is also possible to improve natural products such as natural food and cosmetic products. Therefore, another aspect of the invention is the treatment of natural food products with the the oxidoreductase of the present invention. Examples of such natural food products are extract of fruit, vegetables, foliage, herbs and similar carotene-containing natural products. These may be in further processed form such as in the form of extracts, juices, purees, pulps or the like, such as used in baby food and other processed food, or they may be dried such as dried leaves, petals, fruit, e.g. for use in herbal infusions. The treatment of such natural products with the oxidoreductase will lead to the conversion of the carotenoids naturally occurring on those products, which in turn will improve the flavour and/or fragrance of the food products.

One major advantage of the method of the present invention is that it can be carried out in the absence of any bleach enhancing substances, in particular in the absence of hydrogen peroxide.

The present invention will now be described by way of illustration in the following specific examples, having reference to the accompanying figures, in which:

**Figure 1:** depicts the cDNA sequence of the oxidoreductase derived from *Lepista irina.*

**Figure 2:** depicts the amino acid sequence of the oxidoreductase derived from *Lepista irina* in one-letter code.

**Figure 3:** shows the conversion of β,β-carotene over time (□-carotene: dotted line; □-ionone: drawn through line

**Figure 4:** shows the temperature optimum determination of the oxidoreductase.

**Figure 5:** depicts a GC-chromatogram of the volatile nor-isoprenoids from the conversion of β,β-carotene by the oxidoreductase from *Lepista irina.*

**Figure 6:** (A) shows a photograph of a reference fabric sample stained with carrot juice and treated with medium and a commercially available surfactant (above) and a test fabric sample stained with carrot juice and treated with oxidoreductase of the invention and a commercially available surfactant (below).
(B) shows a photograph of a reference fabric sample stained with carrot juice and treated with medium (above) and a test fabric sample stained with carrot juice and treated with 50μl oxidoreductase of the invention (below).

## Examples

### Example 1

### Isolating the carotene-converting oxidoreductase from *Lepista irina*

The oxidoreductase enzyme from *Lepista irina* was purified from SDS-PAGE gels and partial amino acid sequences were obtained from N-terminal Edman degradation and mass spectrometry (ESI-MSMS).

Primers were deduced from the partial amino acid sequences and a 1274 bp cDNA of the oxidoreductase of *Lepista irina* was sequenced by means of primer walking. By optimisation of the respective annealing temperatures, single PCR bands were produced. The primer walking strategy as well as the PCR primers employed are depicted below:

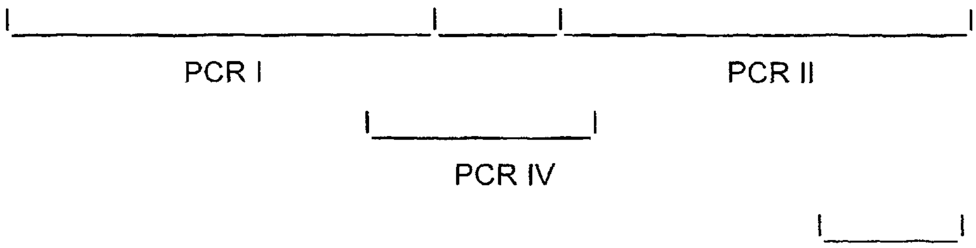


PCR Ia

Lambda fw 2, 6071 E: 5' CGC GCC ATT GTG TTG GTA 3'

pevoxid Lipiv rev, 0690 D: 5' AGC AGT GCC TGG GAA GAG T 3'

PCR IIa

pevoxid Lipiv fw, 0691 D: 5' CCC CAT TGC AAG GAG AGA T 3'

Lambda rev, 0064 E: 5' CGA TGT ACA TGT CGT CAA TGG 3'

PCR III:

perlipend fw, 3646 D: 5' TCC CTG GAT CGT AAA TGC TT 3'

Lambda rev, 0064 E: 5' CGA TGT ACA TGT CGT CAA TGG 3'

PCR IV:

peroxid lipis middle fw, 3721 E: 5' CTC GTG CCA GAG CCT TTT 3'

peroxid lipis middle rev, 3722 E: 5' GGT TCT GAA TCT TCG GTT GG 3'

The sequence obtained comprises an open reading frame (ORF) of 1083 bp, starting from 41 is represented in Figure 1 and SEQ ID NO:1. The ORF encodes an enzyme of 361 amino acids, the sequence of which is depicted in Figure 2 and SEQ ID NO:2.

**Example 2**

**Determining the pH and temperature optimum and iso-electric point of the carotene-converting oxidoreductase from *Lepista irina***

The pH optimum was determined to be 3.6, the temperature optimum was determined to be 34°C. The result is shown in Figure 4.

The iso-electric point was determined by applying retention factors ($R_F$) of standard proteins against their pI-values.

An IEF polyacrylamide gel (Serva, Heidelberg, Germany) of 12.5 cm x 12.5 x 0.3 mm was used, containing an immobilised pH gradient of pH 3 to 10. The samples were subjected to ultrafiltration (Ultrafree 4, cut off 10 kDa, Millipore) at 3300 g and desalted using bidistilled water. They were concentrated to a protein content of 2 mg/ml.

The samples were applicated twice, laterally reversed on both sides of the IEF gel. For detection, the gel was cut concentric and one half each was subjected to coomassie staining and "activity de-staining", respectively. 50 mL of β-carotene solution (0.01 % m/v + surfactant Tween TM 40 1 % m/v), 15 mL buffer solution (7 mM citric acid, 6 mM disodium hydrogen phosphate; pH 3.5), 100 μL trace element solution (containing Fe-, Cu-, Zn-, and Mn-ions), and 0.7 g agarose were mixed to give an orange coloured agarose gel of 2 mm thickness for the de-staining test. One half of the IEF gel was covered with this carotene test agar, pinned down and incubated at 34 °C for 1.5 hours. The active fraction gave a bleaching spot on the test gel. Comparison of the bleaching positions to the sample and reference spots on the coomassie stained gel half allowed the determination of the isoelectric point of the oxidoreductase.

The iso-electric point was determined to be 3.75 for the oxidoreductase of the present invention (not shown).

**Example 3**

**Determination of the molecular weight of the carotene-converting oxidoreductase from *Lepista irina***

The molecular weight was determined by means of gel permeability chromatography (GPC) using a Superdex 200 HR10/30 column, which covers the range of 10-600 kDa. The molecular weight was determined to be about 50 kDa (results not shown).

**Example 4**

**Conversion of β,β-carotene**

Figure 5 shows a GC chromatogram of the volatile nor-isoprenoids from the conversion of β,β-carotene.

The cell-free medium of a culture of *Lepista irina* was concentrated by ultra filtration, mixed with solubilised β,β-carotene and incubated. For emulsification, 20 mg of β,β-carotene and 200 mg of surfactant TweenTM 40 ex Aldrich were dissolved into dichloromethane. The solvent was distilled off under reduced pressure and the residue was resuspended into 50 mL of water.

For the biotransformation, the cell free growth medium of *Lepista irina* was adjusted to pH 3.5 and buffered with citric acid / $Na_2HPO_4$ (1:1, v/v). After addition of 25 mL of the carotene emulsion, the incubation was carried out on a rotary shaker at 150 rpm and 34 °C for 60 minutes. The medium was extracted three times with pentane/dichloromethane (1:1, v/v) and the solution was concentrated to a final volume of 5 mL.

The results are shown in Figure 5 and Table 1. As can be seen, β,β-carotene is converted in high yields to nor-isoprenoids by enzymes obtained from *L.irina.*

Table 1:

| | **Conversions [%]** | **volatile conversion products** |
|---|---|---|
| Retentate *Lepista irina* | **93** | β-ionone, dihydroactinidiolide, β-cyclo-citral 2-hydroxy-2,6,6,-trimethyl-cyclohexanone |
| Permeate *Lepista irina* | **10** | |

**Example 5**

**Kinetic investigations**

The kinetic investigations suggest a rapid reaction profile. The time-dependent decrease of absorption of a β-carotene test solution was monitored at 450 nm using a spectral photometer. A significant decrease of absorption was observable as quickly as 2 minutes after the start of incubation. In biotransformation experiments more than 90% of the initially added β-carotene had been degraded 30 minutes after the start of incubation. The spectrum of the volatile β-carotene conversion products was dominated by the $C_{13}$ compound β-ionone (13 mol-%), indicating an asymmetric (non-centric) cleavage of the $C_{40}$ carotene backbone. Further β-carotene conversion products were 2-hydroxy-2,6,6-trimethyl-cyclohexanone, dihydoractinidiolide, and β-cyclocitral (GC/MS). In accordance with the formation of volatile $C_{13}$ nor-isoprenoids, the corresponding $C_{27}$ apo-carotenal was traceable in LC/MS experiments.

The cell-free medium of a culture of *Lepista irina* was mixed with 2 mg solubilised β,β-carotene and incubated. After 30 minutes, 1 hour, 2 hours, 3 hours and 6 hours, respectively, the reaction was stopped and the mixture extracted.

The result is shown in figure 3. After 30 minutes, the substrate had been almost completely converted. (-□-)

shows the degradation of β,β-carotene, (-□-) the increase in β-ionone. The conversion of α-carotene (yielding α-ionone as the main product), capsanthin, and lycopene proved the extremely broad substrate range of the oxidoreductase.

**Example 6**

**Removal of carrot stains from fabric samples using the oxidoreductase**

*Enzyme preparation:*

One litre of culture medium ($10^{th}$ culture day) was concentrated to 80 ml by ultrafiltration (10 kDa exclusion limit). The total protein concentration was 0.6 mg/ml before and 2.4 mg/ml after the ultrafiltration step. The photometric assay showed good activity of ~75 mU/ml.

*Enzyme assay:*

0.1 ml of an aqueous β,β-carotene solution emulsified with a polyoxyethylene sorbitan ester (Tween™ 40) was added to 1.5 ml of cell-free concentrated growth medium of *Lepista irina.* The time-dependent decrease in absorption was monitored at 450 nm using a spectral photometer. Enzyme activity can be calculated according to the following equation:

$$A\,[Uml^{-1}] = Delta\ E \times V_t / V_s \times d \times e$$

Wherein

| | |
|---|---|
| Delta E | Decrease of absorption per minute |
| $V_t$ | total volume in cuvette [ml] |
| $V_s$ | sample volume in cuvette [ml] |
| d | thickness of cuvette [cm] |
| e | extinction coefficient of β,β-carotene (in water) |

Experimental value: (95000 L $mol^{-1}$ $cm^{-1}$, $\lambda$ = 450 nm)

*Wash performance test on carotenoid stain:*

The pre/post wash test used a pre-wash at specific conditions in each of the Following:

(1) 200 ml demi neutralised water
(2) 200 ml of 1 % solution (surfactant solution) Tween™ 40 made in demi water
(3) 200 ml of 25 mM sodium acetate buffer at pH 3.5 solution

All test solutions contained 10 ppm enzyme. Reference solutions were prepared without enzyme. The pre-wash for the three different treatments were carried out under constant temperature of 28°C for approximately 18 hours, followed by a post-treatment.

Treatments (1) and (2) comprised an extended rinse with tap water, treatment (3) a post-wash in 1% full finished heavy duty liquid detergent (HDL, added to the pre-wash buffer solution) for 15 minutes at 40°C carried out in a Washtec LaunderOmeter. The specific conditions in the pre-wash of treatment (3) are close to the optimal conditions of the enzyme secreted by *Lepista irina.* After treatment, the test fabrics were dried in a tumble dryer.

*Test fabrics:*

Two replicates of carrot stains (3 cm / 3 cm) were made according to the following specifications and used in all treatments:

1. preconditioned knitted cotton (3 x 95°C) with detergent powder without bleach, 1 x 95°C without detergent, tumble dry at the end,

2. Cut the preconditioned fabric into pieces,
3. Soak the pieces in Granini carrot juice and tumble dry,
4. Repeat step 3,
5. Cut homogeneously coloured stained fabric into pieces of 3 cm / 3 cm.

*Visual grading:*

The evaluation was carried out in controlled lighting conditions by visual grading in "panel score units" [psu] of the test fabric versus reference fabrics by expert graders. The units are as follows:

0 = grader sees no difference
1 = graders thinks he sees a difference
2 = grader is certain he sees a difference
3 = grader observes a big difference
4 = grader observes a huge difference

*Results:*

The three different treatments were used with 10 ppm enzyme (total protein) in 200 ml solution in the pre-wash and incubated for approximately 18 hours at 28°C without agitation. The visual grading versus reference in psu are represented in Table 1 below.

Table 1

| **Pre-wash treatment:** 10 ppm enzyme in solution | **Post-wash** | **Visual grading [psu]** Versus nil-enzyme treatment |
|---|---|---|
| In Demi water | Demi water rinse | 0 |
| In 1% Tween™ 40 | Demi water rinse | +1 |
| In buffer ph 3.5 | HDL wash (15 min/40°C) | +3 |

The initial testing showed significant performance on carrot stains of the carotene-specific oxidoreductase of the present invention under optimal conditions (buffer at ph 3.5 at 28°C for approximately 18 hours). An extended soak in 1% Tween TM solution reduced the visual carrot removal. A soak in just water resulted in no visual performance on the stain. See Figure 6 (A) and (B), top fabric sample.

**Example 7**

**Bleaching experiments with carrot stains using the oxidoreductase**

For the bleaching experiment, 50 µl of concentrated culture medium of a culture of *Lepista irina* were applied directly to a carrot stained fabric piece.
To ensure improved bio-availability, a surfactant (TweenTM 40, 1%) was added in a parallel experiment. The samples were incubated at 28°C for 3 and 18 hours, respectively. After the incubation, the samples were rinsed repeatedly with water. For the reference experiments, water was used instead of the concentrated culture medium. All experiments were performed with an older enzyme sample as used in the pre/post wash test as described above.
In this drop test, 50 µl of concentrated culture medium showed significant stain removal and bleaching of the stained area. in contrast, the reference solution (no enzyme) showed no stain removal.
The results are shown in figure 6. The arrow shows the point of application of the oxidoreductase.

**Example 8**

**Examples of detergents used**

The following are examples of detergent. In the detergent compositions, the enzymes levels are expressed by pure enzyme by weight of the total composition and unless otherwise specified, the detergent ingredients are expressed by weight of the total compositions. The abbreviated component identifications therein have the following meanings:

| | |
|---|---|
| LAS : | Sodium linear $C_{11-13}$ alkyl benzene sulphonate. |
| TAS : | Sodium tallow alkyl sulphate. |
| CxyAS : | Sodium $C_{1x}$ - $C_{1y}$ alkyl sulfate. |
| CxySAS : | Sodium $C_{1x}$ - $C_{1y}$ secondary (2,3) alkyl sulfate. |
| MBASx,y : | Sodium mid-chain branched alkyl sulfate having an average of x carbon atoms, whereof an average of y carbon atoms are comprised in (a) branching) unit(s) . |
| CxyEz : | $C_{1x}$ - $C_{1y}$ predominantly linear primary alcohol condensed with an average of z moles of ethylene oxide. |
| CxyEzS : | $C_{1x}$ - $C_{1y}$ sodium alkyl sulfate condensed with an average of z moles of ethylene oxide. |
| CxEOy : | Cy alcohol with an average of ethoxylation of y. |
| Nonionic : | Mixed ethoxylated/propoxylated fatty alcohol e.g. Plurafac LF404 being an alcohol with an average degree of ethoxylation of 3.8 and an average degree of propoxylation of 4.5. |
| QAS : | $R2.N+(CH_3)_2(C_2H_4OH)$ with $R_2 = C_{12}-C_{14}$. |
| SADS : | Sodium C14-22 alkyl disulfate of the formula 2-R.C4H7.-1,4-(SO4-)2 where R = C10-18. |
| MES : | x-sulpho methyl ester of C18 fatty acid. |
| Soap : | Sodium linear alkyl carboxylate derived from a 80/20 mixture of tallow and coconut fatty acids. |
| Silicate : | Amorphous Sodium Silicate ($SiO_2$:$Na_2O$ ratio = 1.6-3.2:1). |
| Metasilicate : | Sodium metasilicate ($SiO_2$:$Na_2O$ ratio = 1.0). |
| Zeolite A : | Hydrated Sodium Aluminosilicate of formula $Na_{12}(AlO_2SiO_2)_{12}$. $27H_2O$ having a primary particle size in the range from 0.1 to 10 micrometers (Weight expressed on an anhydrous basis). |
| (Na-)SKS-6 : | Crystalline layered silicate of formula $\delta$-$Na_2Si_2O_5$. |
| Citrate : | Tri-sodium citrate dihydrate. |
| Citric : | Anhydrous citric acid. |
| Carbonate : | Anhydrous sodium carbonate. |
| Bicarbonate : | Sodium hydrogen carbonate. |
| Sulphate : | Anhydrous sodium sulphate. |
| STPP : | Sodium tripolyphosphate. |
| TSPP : | Tetrasodium pyrophosphate. |
| MA/AA : | Random copolymer of 4:1 acrylate/maleate, average molecular weight about 70,000-80,000. |
| MA/AA 1 : | Random copolymer of 6:4 acrylate/maleate, average molecular weight about 10,000. |
| AA : | Sodium polyacrylate polymer of average molecular weight 4,500. |
| Polycarboxylate : | Copolymer comprising mixture of carboxylated monomers such as acrylate, maleate and methyacrylate with a MW ranging between 2,000-80,000 such as Sokolan commercially available from BASF, being a copolymer of acrylic acid, MW4,500. |
| BB1 : | 3-(3,4-Dihydroisoquinolinium)propane sulfonate as prepared in the preparation example 1 |
| BB2 | 1-(3,4-dihydroisoquinolinium)-decane-2-sulfate as prepared in the preparation example 2 |
| PB1 : | Anhydrous sodium perborate monohydrate. |
| PB4 : | Sodium perborate tetrahydrate of nominal formula $NaBO_3.4H_2O$. |
| Percarbonate : | Anhydrous sodium percarbonate of nominal formula 2.74 $Na_2CO_3.3H_2O_2$ . |
| DAP 1 | Diacyl Peroxide Particle with 30% dibenzoyl peroxide, 40% sodium sulfate, 5% Acusol 480N polymer, 2% maltodextrin, 12% ethoxylated stearyl alcohol, and balance as water. |
| DAP 2 | Dilauroyl peroxide available from Akzo |
| NaDCC : | Sodium dichloroisocyanurate. |
| TAED : | Tetraacetyl ethylene diamine. |
| NOBS : | Nonanoyloxybenzene sulfonate in the form of the sodium salt. |
| NACA-OBS : | (6-nonamidocaproyl) oxybenzene sulfonate. |
| DOBS : | Decanoyl oxybenzene sulfonate in the form of the sodium salt. |
| DTPA : | Diethylene triamine pentaacetic acid. |
| HEDP : | 1,1-hydroxyethane diphosphonic acid. |
| DETPMP : | Diethyltriamine penta (methylene) phosphonate, marketed by Monsanto under the Trade name Dequest 2060. |
| EDDS : | Ethylenediamine-N,N'-disuccinic acid, (S,S) isomer in the form of its sodium salt |
| Chelant : | Chelant selected from EEDS, HEDP, DTPA, DETPMP and/or mixtures thereof. |
| Catalyst | Mn(bycyclam)Cl2 |
| MnTACN : | Manganese 1,4,7-trimethyl-1,4,7-triazacyclononane. |
| Photoactivated : Bleach | Sulfonated zinc phtalocyanine encapsulated in dextrin soluble polymer. |

| | |
|---|---|
| Photoactivated : Bleach 1 | Sulfonated alumino phtalocyanine encapsulated in dextrin soluble polymer. |
| PAAC : | Pentaamine acetate cobalt(III) salt. |
| Paraffin : | Paraffin oil sold under the tradename Winog 70 by Wintershall. |
| NaBz : | Sodium benzoate. |
| Oxidoreductase of : | Carotene-degrading oxidoreductase from *Lepista irina* the present invention according to the present invention |
| Protease : | Proteolytic enzyme sold under the tradename Savinase , Alcalase, Durazym by Novo Nordisk A/S, Maxacal, Maxapem sold by Gist-Brocades and proteases described in patents WO91/06637 and/or WO95/10591 and/or EP 251 446. |
| Amylase : | Amylolytic enzyme sold under the tradename Purafact Ox Am$^R$ described in WO 94/18314, WO96/05295 sold by Genencor; Termamyl® , Fungamyl® and Duramyl® , all available from Novo Nordisk A/S and those described in WO95/26397 (sold under the tradename Natalase By Novo Nordisk). |
| Lipase : | Lipolytic enzyme sold under the tradename Lipolase Lipolase Ultra by Novo Nordisk A/S and Lipomax by Gist-Brocades. |
| Cellulase : | Cellulytic enzyme sold under the tradename Carezyme, Celluzyme and/or Endolase by Novo Nordisk A/S. |
| CMC : | Sodium carboxymethyl cellulose. |
| PVNO : | Polyvinylpyridine-N-Oxide, with an average molecular weight of 50,000. |
| PVPVI : | Copolymer of vinylimidazole and vinylpyrrolidone, with an average molecular weight of 20,000. |
| Brightener 1 : | Disodium 4,4'-bis(2-sulphostyryl)biphenyl. |
| Brightener 2 : | Disodium 4,4'-bis(4-anilino-6-morpholino-1.3.5-triazin-2-yl) stilbene-2:2'-disulfonate. |
| Silicone antifoam : | Polydimethylsiloxane foam controller with siloxane-oxyalkylene copolymer as dispersing agent with a ratio of said foam controller to said dispersing agent of 10:1 to 100:1. |
| Suds Suppressor : | 12% Silicone/silica, 18% stearyl alcohol,70% starch in granular form. |
| Thickener : | High molecular weight crosslinked polyacrylates such as Carbopol offered by B.F. Goodrich Chemical Company and Polygel. |
| SRP 1 : | Anionically end capped poly esters. |
| QEA : | $bis((C_2H_5O)(C_2H_4O)_n)(CH_3)$ $-N^+-C_6H_{12}-N^+-(CH_3)$ $bis((C_2H_5O)-(C_2H_4O))_n$, wherein n = from 20 to 30. |
| PEGX : | Polyethylene glycol,of a molecular weight of x. |
| PEO : | Polyethylene oxide, with an average molecular weight of 5,000. |
| TEPAE : | Tetreaethylenepentaamine ethoxylate. |
| BTA : | Benzotriazole. |
| pH : | Measured as a 1 % solution in distilled water at 20°C. |

**Example 9**

The following high density and bleach-containing laundry detergent compositions were prepared according to the present invention:

| | I | II | III | IV | V | VI |
|---|---|---|---|---|---|---|
| Blown Powder | | | | | | |
| Zeolite A | 12.0 | - | 15.0 | 12.0 | - | 15.0 |
| Sulfate | - | 5.0 | - | - | 5.0 | - |
| LAS | 3.0 | - | 3.0 | 3.0 | - | 3.0 |
| C45AS | 3.0 | 2.0 | 4.0 | 3.0 | 2.0 | 4.0 |
| QAS | - | - | 1.5 | - | - | 1.5 |
| DETPMP | 0.4 | 0.4 | 0.4 | 0.4 | 0.4 | 0.4 |
| CMC | 0.4 | 0.4 | 0.4 | 0.4 | 0.4 | 0.4 |
| MA/AA | 1.0 | 2.0 | 2.0 | 1.0 | 2.0 | 2.0 |
| Agglomerates | | | | | | |
| QAS | 1.0 | - | - | 1.0 | - | - |
| LAS | - | 11.0 | 7.0 | - | 11.0 | 7.0 |

(continued)

| | I | II | III | IV | V | VI |
|---|---|---|---|---|---|---|
| Agglomerates | | | | | | |
| TAS | 2.0 | 2.0 | 1.0 | 2.0 | 2.0 | 1.0 |
| Silicate | 3.0 | - | 4.0 | 3.0 | - | 4.0 |
| Zeolite A | 8.0 | 8.0 | 8.0 | 8.0 | 8.0 | 8.0 |
| Carbonate | 8.0 | 8.0 | 4.0 | 8.0 | 8.0 | 4.0 |
| Agglomerate | | | | | | |
| NaSKS-6 | 15.0 | 12.0 | 5.0 | 15.0 | 12.0 | 5.0 |
| LAS | 8.0 | 7.0 | 4.0 | 8.0 | 7.0 | 4.0 |
| Spray On | | | | | | |
| Perfume | 0.3 | 0.3 | 0.3 | 0.3 | 0.3 | 0.3 |
| C25E3 | 2.0 | - | 2.0 | 2.0 | - | 2.0 |
| Dry additives | | | | | | |
| QEA | 1.0 | 0.5 | 0.5 | 1.0 | 0.5 | 0.5 |
| Citric/Citrate | 5.0 | - | 2.0 | 5.0 | - | 2.0 |
| Bicarbonate | - | 3.0 | - | - | 3.0 | - |
| Carbonate | 8.0 | 15.0 | 10.0 | 8.0 | 15.0 | 10.0 |
| TAED and/ or | 6.0 | - | 5.0 | 6.0 | - | 5.0 |
| NACA-OBS | | | | | | |
| NOBS | - | 2.0 | - | - | 2.0 | - |
| DAP 1 | - | - | - | 6.7 | 4.8 | 5.2 |
| Catalyst | 0.002 | - | 0.02 | - | 0.02 | - |
| Percarbonate or PB1 | 14.0 | 7.0 | 10.0 | 4.15 | 7.0 | 10.0 |
| BB1 | 0.40 | - | 0.20 | - | - | - |
| BB2 | - | 0.14 | - | - | - | - |
| Polyethylene oxide of MW 5,000,000 | - | - | 0.2 | - | - | 0.2 |
| Bentonite clay | - | - | 10.0 | - | - | 10.0 |
| Citric acid | 4.0 | - | 1.5 | 4.0 | - | 1.5 |
| Oxidoreductase of the present invention | 0.001 | 0.02 | 0.01 | 0.001 | 0.02 | 0.01 |
| Protease | 0.033 | 0.033 | 0.033 | 0.033 | 0.033 | 0.033 |
| Lipase | 0.008 | 0.008 | 0.008 | 0.008 | 0.008 | 0.008 |
| Amylase | 0.001 | 0.001 | 0.001 | 0.001 | 0.001 | 0.001 |
| Cellulase | 0.0014 | 0.0014 | 0.0014 | 0.0014 | 0.0014 | 0.0014 |
| Silicone antifoam | 5.0 | 5.0 | 5.0 | 5.0 | 5.0 | 5.0 |
| Sulfate | - | 3.0 | - | - | 3.0 | - |
| Density (g/litre) | 850 | 850 | 850 | 850 | 850 | 850 |
| Moisture and miscellaneous | Up to 100% | | | | | |

**Example 10**

The following laundry compositions, which may be in the form of granules or tablet, were prepared according to the present invention.

| | I | II | III | IV | V |
|---|---|---|---|---|---|
| Base Product | | | | | |
| C45 AS/TAS | 8.0 | 5.0 | 3.0 | 3.0 | 3.0 |

(continued)

| | I | II | III | IV | V |
|---|---|---|---|---|---|
| Base Product | | | | | |
| LAS | 8.0 | - | 8.0 | - | 7.0 |
| C25AE3S | 0.5 | 2.0 | 1.0 | - | - |
| C25AE5/AE3 | 2.0 | - | 5.0 | 2.0 | 2.0 |
| QAS | - | - | - | 1.0 | 1.0 |
| Zeolite A | 20.0 | 18.0 | 11.0 | - | 10.0 |
| SKS-6 (I) (dry add) | - | - | 9.0 | - | - |
| MA/AA | 2.0 | 2.0 | 2.0 | - | - |
| AA | - | - | - | - | 4.0 |
| Citrate | - | 2.0 | - | - | - |
| Citric | 2.0 | - | 1.5 | 2.0 | - |
| DTPA | 0.2 | 0.2 | - | - | - |
| EDDS | - | - | 0.5 | 0.1 | - |
| HEDP | - | - | 0.2 | 0.1 | - |
| PB1 | 3.0 | 5.0 | 10.0 | - | 4.0 |
| Percarbonate | - | - | - | 18.0 | - |
| NOBS | 3.0 | 4.0 | - | - | 4.0 |
| NACA OBS | - | - | 2.0 | - | - |
| TAED | - | - | 2.0 | 5.0 | - |
| BB1 | 0.06 | - | 0.34 | - | 0.14 |
| BB2 | - | 0.14 | - | 0.20 | - |
| Catalyst | - | 0.001 | - | - | 0.002 |
| Carbonate | 15.0 | 18.0 | 8.0 | 15.0 | 15.0 |
| Sulphate | 5.0 | 12.0 | 2.0 | 17.0 | 3.0 |
| Silicate | - | 1.0 | - | - | 8.0 |
| Protease | 0.033 | 0.033 | 0.033 | 0.046 | 0.033 |
| Lipase | 0.008 | 0.008 | 0.008 | 0.008 | 0.006 |
| Amylase | 0.001 | 0.001 | 0.001 | 0.0014 | 0.001 |
| Cellulase | 0.0014 | 0.0014 | 0.0014 | 0.01 | - |
| Oxidoreductase of the present invention | 0.001 | 0.002 | 0.02 | 0.05 | 0.005 |
| Minors | 0.5 | 0.5 | 0.5 | 0.5 | 0.5 |
| Perfume | 0.2 | 0.3 | 0.5 | 0.2 | 0.1 |
| Moisture and miscellaneous | Up to 100% | | | | |

Minors include Brightener / SRP1 / CMC / Photobleach / MgSO4 / PVPVI/ Suds suppressor /PEG.

**Example 11**

The following granular detergent were prepared in accordance with the present invention:

| | I | II | III | IV | V | VI | VII | VIII |
|---|---|---|---|---|---|---|---|---|
| Base granule | | | | | | | | |
| STPP | - | 22.0 | - | 15.0 | - | 22.0 | - | 15.0 |
| Zeolite A | 30.0 | - | 24.0 | 5.0 | 30.0 | - | 24.0 | 5.0 |
| Sulfate | 5.5 | 5.0 | 7.0 | 7.0 | 5.5 | 5.0 | 7.0 | 7.0 |
| MA/AA | 3.0 | - | - | - | 3.0 | - | - | - |
| AA | - | 1.6 | 2.0 | - | - | 1.6 | 2.0 | - |

(continued)

| | I | II | III | IV | V | VI | VII | VIII |
|---|---|---|---|---|---|---|---|---|
| Base granule | | | | | | | | |
| MA/AA (1) | - | 12.0 | - | 6.0 | - | 12.0 | - | 6. 0 |
| LAS | 14.0 | 10.0 | 9.0 | 20.0 | 14.0 | 10.0 | 9.0 | 20.0 |
| C45AS | 8.0 | 7.0 | 9.0 | 7.0 | 8.0 | 7.0 | 9.0 | 7.0 |
| C45AE11 S | - | 1.0 | - | 1.0 | - | 1.0 | - | 1.0 |
| MES | 0.5 | 4.0 | 6.0 | - | 0.5 | 4.0 | 6.0 | - |
| SADS | 2.5 | - | - | 1.0 | 2.5 | - | - | 1.0 |
| Silicate | - | 1.0 | 0.5 | 10.0 | - | 1.0 | 0.5 | 10.0 |
| Soap | - | 2.0 | - | - | - | 2.0 | - | - |
| Brightener 1 | 0.2 | 0.2 | 0.2 | 0.2 | 0.2 | 0.2 | 0.2 | 0.2 |
| Carbonate | 6.0 | 9.0 | 8.0 | 10.0 | 6.0 | 9.0 | 8.0 | 10.0 |
| PEG 4000 | - | 1.0 | 1.5 | - | - | 1.0 | 1.5 | - |
| DTPA | - | 0.4 | - | - | - | 0.4 | - | - |
| Spray on | | | | | | | | |
| C25E9 | - | - | - | 5.0 | - | - | - | 5.0 |
| C45E7 | 1.0 | 1.0 | - | - | 1.0 | 1.0 | - | - |
| C23E9 | - | 1.0 | 2.5 | - | - | 1.0 | 2.5 | - |
| Perfume | 0.2 | 0.3 | 0.3 | - | 0.2 | 0.3 | 0.3 | - |
| Dry additives | | | | | | | | |
| Carbonate | 5.0 | 10.0 | 13.0 | 8.0 | 5.0 | 10.0 | 13.0 | 8.0 |
| PVPVI/PVNO | 0.5 | - | 0.3 | - | 0.5 | - | 0.3 | - |
| Protease | 0.033 | 0.033 | 0.033 | .0016 | 0.033 | 0.033 | 0.033 | .0016 |
| Lipase | 0.008 | - | - | 0.008 | 0.008 | - | - | 0.008 |
| Amylase | .0016 | - | - | .0016 | .0016 | - | - | .0016 |
| Cellulase | .0002 | .0005 | .0005 | .0002 | .0002 | .0005 | .0005 | .0002 |
| Oxidoreductase of the present invention | 0.001 | 0.02 | 0.03 | 0.015 | 0.001 | 0.02 | 0.03 | 0.015 |
| DTPA | 0.5 | 0.3 | 0.5 | 1.0 | 0.5 | 0.3 | 0.5 | 1.0 |
| PB1 | 5 | 3.0 | 10 | 4.0 | 5 | 3.0 | 10 | 4.0 |
| DAP 1 | - | - | - | - | 3.8 | 6.7 | 4.3 | 3.2 |
| Catalyst | 0.001 | - | - | 0.002 | - | 0.001 | - | - |
| BB1 | 0.2 | - | - | 0.5 | - | - | - | - |
| BB2 | - | 0.3 | 0.4 | - | - | - | - | - |
| NOBS/TAED | 0.5 | 0.3 | 0.5 | 0.6 | 0.5 | 0.3 | 0.5 | 0.6 |
| Sulfate | 4.0 | 5.0 | - | 5.0 | 4.0 | 5.0 | - | 5.0 |
| SRP1 | - | 0.4 | - | - | - | 0.4 | - | - |
| Sud supressor | - | 0.5 | - | - | - | 0.5 | - | - |
| speckle | 0.9 | - | 2.7 | 1.2 | 0.9 | - | 2.7 | 1.2 |
| Moisture and miscellaneous | Up to 100% | | | | | | | |

**Example 12**

The following laundry detergent compositions were prepared in accordance with the present invention:

| | I | II | III | IV | V | VI | VII | VIII |
|---|---|---|---|---|---|---|---|---|
| LAS | 13.3 | 13.7 | 10.4 | 8.0 | 13.3 | 13.7 | 10.4 | 8.0 |
| $C_{45}$ AS | 3.9 | 4.0 | 4.5 | - | 3.9 | 4.0 | 4.5 | - |
| $C_{45}$ E0.5S | 2.0 | 2.0 | - | - | 2.0 | 2.0 | - | - |

(continued)

| | I | II | III | IV | V | VI | VII | VIII |
|---|---|---|---|---|---|---|---|---|
| C45 E3S | - | - | - | - | - | - | - | - |
| C45E6.5S | 0.5 | 0.5 | 0.5 | 5.0 | 0.5 | 0.5 | 0.5 | 5.0 |
| $C_9$-$C_{14}$ alkyl dimethyl hydroxy ethyl quaternary NH4 salt | 1.0 | - | - | 0.5 | 1.0 | - | - | 0.5 |
| Tallow fatty acid | 0.5 | - | - | - | 0.5 | - | - | - |
| Tallow alcohol - ethoxylate (50) | | - | 1.0 | 0.3 | - | - | 1.0 | 0.3 |
| STPP | - | 41.0 | - | 20.0 | - | 41.0 | - | 20.0 |
| Zeolite A | 26.3 | - | 21.3 | 1.0 | 26.3 | - | 21.3 | 1.0 |
| Carbonate | 23.9 | 12.4 | 25.2 | 17.0 | 23.9 | 12.4 | 25.2 | 17.0 |
| Sodium Polyacrylate (45%) | 3.4 | 0.0 | 2.7 | - | 3.4 | 0.0 | 2.7 | - |
| MA/AA | - | - | 1.0 | 1.5 | - | - | 1.0 | 1.5 |
| Silicate (1:6 ratio) | 2.4 | 6.4 | 2.1 | 6.0 | 2.4 | 6.4 | 2.1 | 6.0 |
| Sulfate | 10.5 | 10.9 | 8.2 | 15.0 | 10.5 | 10.9 | 8.2 | 15.0 |
| PB1 | 1.0 | 1.0 | 1.0 | 2.0 | 1.0 | 1.0 | 1.0 | 2.0 |
| PEG MW 4000 (50%) | 1.7 | 0.4 | 1.0 | - | 1.7 | 0.4 | 1.0 | - |
| CMC | 1.0 | - | - | 0.3 | 1.0 | - | - | 0.3 |
| Citric | - | - | 3.0 | - | - | - | 3.0 | - |
| BB1 | 1.0 | 0.5 | 0.6 | - | - | - | - | - |
| BB2 | - | 0.2 | - | 1.0 | - | - | - | - |
| DAP 1 | - | - | - | - | 2.0 | 2.1 | 3.4 | 2.1 |
| NOBS/ DOBS | 0.2 | 0.5 | 0.5 | 0.1 | - | - | - | - |
| TAED | 0.6 | 0.5 | 0.4 | 0.3 | - | - | - | - |
| SRP 1 | 1.5 | - | - | - | 1.5 | - | - | - |
| SRP2 | - | 1.5 | 1.0 | 1.0 | - | 1.5 | 1.0 | 1.0 |
| Moisture | 7.5 | 3.1 | 6.1 | 7.3 | 7.5 | 3.1 | 6.1 | 7.3 |
| Mn sulphate | - | - | - | 1.0 | - | - | - | 1.0 |
| Chelant | - | - | - | 0.5 | - | - | - | 0.5 |
| speckles | 0.5 | 1.0 | 3.0 | 0.5 | 0.5 | 1.0 | 3.0 | 0.5 |
| Protease | 0.033 | 0.033 | 0.033 | 0.046 | 0.033 | 0.033 | 0.033 | 0.046 |
| Lipase | 0.008 | 0.008 | 0.008 | 0.008 | 0.008 | 0.008 | 0.008 | 0.008 |
| Amylase | 0.001 | 0.001 | 0.001 | .0014 | 0.001 | 0.001 | 0.001 | .0014 |
| Cellulase | .0014 | .0014 | .0014 | 0.01 | .0014 | .0014 | .0014 | 0.01 |
| Oxidoreductase of the present invention | 0.001 | 0.01 | 0.005 | 0.002 | 0.001 | 0.01 | 0.005 | 0.002 |
| Minors | 1.0 | 1.0 | 1.0 | 1.0 | 1.0 | 1.0 | 1.0 | 1.0 |

**Example 13**

The following granular fabric detergent compositions which provide "softening through the wash" capability were prepared according to the present invention :

| | I | II | III | IV |
|---|---|---|---|---|
| C45AS | - | 10.0 | - | 10.0 |
| LAS | 7.6 | - | 7.6 | - |
| C68AS | 1.3 | - | 1.3 | - |
| C45E7 | 4.0 | - | 4.0 | - |
| C25E3 | - | 5.0 | - | 5.0 |
| Coco-alkyl-dimethyl hydroxyethyl ammonium chloride | 1.4 | 1.0 | 1.4 | 1.0 |
| Citrate | 5.0 | 3.0 | 5.0 | 3.0 |
| Na-SKS-6 | - | 11.0 | - | 11.0 |

(continued)

| | I | II | III | IV |
|---|---|---|---|---|
| Zeolite A | 15.0 | 15.0 | 15.0 | 15.0 |
| MA/AA | 4.0 | 4.0 | 4.0 | 4.0 |
| DETPMP | 0.4 | 0.4 | 0.4 | 0.4 |
| DAP 1 | 4.8 | 6.7 | - | - |
| Catalyst | - | - | 0.001 | 0.001 |
| Percarbonate | - | - | - | 15.0 |
| PB1 | - | - | 15.0 | - |
| TAED | - | - | 5.0 | 5.0 |
| Smectite clay | 10.0 | 10.0 | 10.0 | 10.0 |
| HMWPEO | - | 0.1 | - | 0.1 |
| Oxidoreductase of the present invention | 0.001 | 0.01 | 0.001 | 0.01 |
| Protease | 0.02 | 0.01 | 0.02 | 0.01 |
| Lipase | 0.02 | 0.01 | 0.02 | 0.01 |
| Amylase | 0.03 | 0.005 | 0.03 | 0.005 |
| Cellulase | 0.001 | - | 0.001 | - |
| Silicate | 3.0 | 5.0 | 3.0 | 5.0 |
| Carbonate | 10.0 | 10.0 | 10.0 | 10.0 |
| Suds suppressor | 1.0 | 4.0 | 1.0 | 4.0 |
| CMC | 0.2 | 0.1 | 0.2 | 0.1 |
| Miscellaneous and minors | Up to 100% | | | |

**Example 14**

The following liquid detergent formulations were prepared according to the present invention (Levels are given in parts per weight, enzyme are expressed in pure enzyme) :

| | I | II | III | IV | V |
|---|---|---|---|---|---|
| LAS | 11.5 | 9.0 | - | 4.0 | - |
| C25E2.5S | - | 3.0 | 18.0 | - | 16.0 |
| C45E2.25S | 11.5 | 3.0 | - | 16.0 | - |
| C23E9 | - | 3.0 | 2.0 | 2.0 | 1.0 |
| C23E7 | 3.2 | - | - | - | - |
| CFAA | - | - | 5.0 | - | 3.0 |
| TPKFA | 2.0 | - | 2.0 | 0.5 | 2.0 |
| Citric (50%) | 6.5 | 1.0 | 2.5 | 4.0 | 2.5 |
| Ca formate | 0.1 | 0.06 | 0.1 | - | - |
| Na formate | 0.5 | 0.06 | 0.1 | 0.05 | 0.05 |
| SCS | 4.0 | 1.0 | 3.0 | 1.2 | - |
| Borate | 0.6 | - | 3.0 | 2.0 | 3.0 |
| Na hydroxide | 6.0 | 2.0 | 3.5 | 4.0 | 3.0 |
| Ethanol | 2.0 | 1.0 | 4.0 | 4.0 | 3.0 |
| 1,2 Propanediol | 3.0 | 2.0 | 8.0 | 8.0 | 5.0 |
| Monoethanolamine | 3.0 | 1.5 | 1.0 | 2.5 | 1.0 |
| TEPAE | 2.0 | - | 1.0 | 1.0 | 1.0 |
| Catalyst | 0.01 | 0.01 | 0.005 | 0.005 | 0.1 |
| Oxidoreductase of the present invention | 0.001 | 0.002 | 0.01 | 0.01 | 0.005 |
| Protease | 0.03 | 0.01 | 0.03 | 0.02 | 0.02 |
| Lipase | - | - | 0.002 | - | - |
| Amylase | - | - | - | 0.002 | - |

(continued)

| | I | II | III | IV | V |
|---|---|---|---|---|---|
| Cellulase | - | - | 0.0002 | 0.0005 | 0.0001 |
| SRP 1 | 0.2 | - | 0.1 | - | - |
| DTPA | - | - | 0.3 | - | - |
| PVNO | - | - | 0.3 | - | 0.2 |
| Brightener 1 | 0.2 | 0.07 | 0.1 | - | - |
| Silicone antifoam | 0.04 | 0.02 | 0.1 | 0.1 | 0.1 |
| Miscellaneous and water | | | | | |

**Example 15**

The following laundry bar detergent compositions were prepared according to the present invention (Levels are given in parts per weight, enzyme are expressed in pure enzyme) :

| | I | II | III | VI | V | III | VI | V |
|---|---|---|---|---|---|---|---|---|
| LAS | - | - | 19.0 | 15.0 | 21.0 | 6.75 | 8.8 | - |
| C28AS | 30.0 | 13.5 | - | - | - | 15.75 | 11.2 | 22.5 |
| Na Laurate | 2.5 | 9.0 | - | - | - | - | - | - |
| Zeolite A | 2.0 | 1.25 | - | - | - | 1.25 | 1.25 | 1.25 |
| Carbonate | 20.0 | 3.0 | 13.0 | 8.0 | 10.0 | 15.0 | 15.0 | 10.0 |
| Ca Carbonate | 27.5 | 39.0 | 35.0 | - | - | 40.0 | - | 40.0 |
| Sulfate | 5.0 | 5.0 | 3.0 | 5.0 | 3.0 | - | - | 5.0 |
| TSPP | 5.0 | - | - | - | - | 5.0 | 2.5 | - |
| STPP | 5.0 | 15.0 | 10.0 | - | - | 7.0 | 8.0 | 10.0 |
| Bentonite clay | - | 10.0 | - | - | 5.0 | - | - | - |
| DETPMP | - | 0.7 | 0.6 | - | 0.6 | 0.7 | 0.7 | 0.7 |
| CMC | - | 1.0 | 1.0 | 1.0 | 1.0 | - | - | 1.0 |
| Talc | - | - | 10.0 | 15.0 | 10.0 | - | - | - |
| Silicate | - | - | 4.0 | 5.0 | 3.0 | - | - | - |
| PVNO | 0.02 | 0.03 | - | 0.01 | - | 0.02 | - | - |
| MA/AA | 0.4 | 1.0 | - | - | 0.2 | 0.4 | 0.5 | 0.4 |
| SRP 1 | 0.3 | 0.3 | 0.3 | 0.3 | 0.3 | 0.3 | 0.3 | 0.3 |
| Oxidoreductase of the present invention | 0.01 | 0.001 | 0.005 | 0.02 | 0.02 | 0.001 | 0.01 | 0.01 |
| Amylase | - | - | 0.01 | - | - | - | 0.002 | - |
| Protease | - | 0.004 | - | 0.003 | 0.003 | - | - | 0.003 |
| Lipase | - | 0.002 | - | 0.002 | - | - | - | - |
| Cellulase | - | .0003 | - | - | .0003 | .0002 | - | - |
| Catalyst | 1.0 | 5.0 | 0.1 | 3.0 | 10.0 | 1.0 | 0.3 | 1.0 |
| PEO | - | 0.2 | - | 0.2 | 0.3 | - | - | 0.3 |
| Perfume | 1.0 | 0.5 | 0.3 | 0.2 | 0.4 | - | - | 0.4 |
| Mg sulfate | - | - | 3.0 | 3.0 | 3.0 | - | - | - |
| Brightener | 0.15 | 0.1 | 0.15 | - | - | - | - | 0.1 |
| Photoactivated bleach (ppm) | - | 15.0 | 15.0 | 15.0 | 15.0 | - | - | 15.0 |

**Example 16**

The following laundry bar detergent compositions were prepared according to the present invention (Levels are given in parts per weight, enzyme are expressed in pure enzyme) :

| | I | II | III | IV | V | VI | VII | VIII |
|---|---|---|---|---|---|---|---|---|
| LAS | - | - | 19.0 | 15.0 | 21.0 | 6.75 | 8.8 | - |
| C28AS | 30.0 | 13.5 | - | - | - | 15.75 | 11.2 | 22.5 |
| Na Laurate | 2.5 | 9.0 | - | - | - | - | - | - |
| Zeolite A | 2.0 | 1.25 | - | - | - | 1.25 | 1.25 | 1.25 |
| Carbonate | 8.0 | 3.0 | 1.0 | 8.0 | 10.0 | 15.0 | 3.0 | 10.0 |
| Ca Carbonate | 27.5 | 27.0 | 35.0 | - | - | 28.0 | - | 28.0 |
| Sulfate | 5.0 | 5.0 | 3.0 | 5.0 | 3.0 | - | - | 5.0 |
| TSPP | 5.0 | - | - | - | - | 5.0 | 2.5 | - |
| STPP | 5.0 | 15.0 | 10.0 | - | - | 7.0 | 8.0 | 10.0 |
| Bentonite clay | - | 10.0 | - | - | 5.0 | - | - | - |
| DETPMP | - | 0.7 | 0.6 | - | 0.6 | 0.7 | 0.7 | 0.7 |
| CMC | - | 1.0 | 1.0 | 1.0 | 1.0 | - | - | 1.0 |
| Talc | - | - | 10.0 | 15.0 | 10.0 | - | - | - |
| Silicate | - | - | 4.0 | 5.0 | 3.0 | - | - | - |
| PVNO | 0.02 | 0.03 | - | 0.01 | - | 0.02 | - | - |
| MA/AA | 0.4 | 1.0 | - | - | 0.2 | 0.4 | 0.5 | 0.4 |
| SRP 1 | 0.3 | 0.3 | 0.3 | 0.3 | 0.3 | 0.3 | 0.3 | 0.3 |
| Oxidoreductas e of the present invention | 0.01 | 0.001 | 0.005 | 0.02 | 0.02 | 0.1 | 0.01 | 0.01 |
| Amylase | - | - | 0.01 | - | - | - | 0.002 | - |
| Protease | - | 0.004 | - | 0.003 | 0.003 | - | - | 0.003 |
| Lipase | - | 0.002 | - | 0.002 | - | - | - | - |
| Cellulase | - | .0003 | - | - | .0003 | .0002 | - | - |
| PEO | - | 0.2 | - | 0.2 | 0.3 | - | - | 0.3 |
| Perfume | 1.0 | 0.5 | 0.3 | 0.2 | 0.4 | - | - | 0.4 |
| Mg sulfate | - | - | 3.0 | 3.0 | 3.0 | - | - | - |
| Brightener | 0.15 | 0.1 | 0.15 | - | - | - | - | 0.1 |
| Catalyst | 0.001 | - | - | 0.001 | - | - | - | - |
| BB1 | 0.2 | 0.2 | 0.3 | - | - | 0.4 | - | - |
| BB2 | - | - | - | 0.4 | 0.5 | - | 0.45 | 0.3 |
| TAED | 1.7 | 1.7 | 1.7 | 1.7 | 1.7 | 1.7 | 1.7 | 1.7 |
| PB4 | 12.0 | 12.0 | 12.0 | 12.0 | 12.0 | 12.0 | 12.0 | 12.0 |
| NOBS | 0.2 | 0.2 | 0.2 | 0.20 | 0.2 | 0.2 | 0.2 | 0.2 |
| Photoactivated | - | 15.0 | 15.0 | 15.0 | 15.0 | - | - | 15.0 |

**Example 17**

The following compact high density (0.96Kg/l) dishwashing detergent compositions were prepared according to the present invention :

| | I | II | III | IV | V | VI |
|---|---|---|---|---|---|---|
| STPP | - | 51.0 | 51.0 | - | - | 44.3 |
| Citrate | 17.0 | - | - | 50.0 | 40.2 | - |
| Carbonate | 17.5 | 14.0 | 20.0 | - | 8.0 | 33.6 |
| Bicarbonate | - | - | - | 26.0 | - | - |
| Silicate | 15.0 | 15.0 | 8.0 | - | 25.0 | 3.6 |
| Metasilicate | 2.5 | 4.5 | 4.5 | - | - | - |
| PB1 | 10.0 | 8.0 | 8.0 | - | - | - |
| PB4 | - | - | - | 10.0 | - | - |
| Percarbonate | - | - | - | - | 11.8 | 4.8 |
| BB1 | - | 0.1 | 0.1 | - | 0.5 | - |

(continued)

| | I | II | III | IV | V | VI |
|---|---|---|---|---|---|---|
| BB2 | 0.2 | 0.05 | - | 0.1 | - | 0.6 |
| Nonionic | 2.0 | 1.5 | 1.5 | 3.0 | 1.9 | 5.9 |
| TAED | 2.0 | - | - | 4.0 | - | 1.4 |
| HEDP | 1.0 | - | - | - | - | - |
| DETPMP | 0.6 | - | - | - | - | - |
| MnTACN | - | - | - | - | 0.01 | - |
| PAAC | - | 0.01 | 0.01 | - | - | - |
| Paraffin | 0.5 | 0.4 | 0.4 | 0.6 | - | - |
| Oxidoreductase of the present invention | 0.04 | 0.1 | 0.03 | 0.5 | 0.005 | 0.005 |
| Protease | 0.072 | 0.053 | 0.053 | 0.026 | 0.059 | 0.01 |
| Amylase | 0.012 | 0.012 | 0.012 | 0.021 | 0.021 | 0.006 |
| Lipase | - | 0.001 | - | 0.005 | - | - |
| BTA | 0.3 | 0.2 | 0.2 | 0.3 | 0.3 | 0.3 |
| Polycarboxylate | 6.0 | - | - | - | 4.0 | 0.9 |
| Perfume | 0.2 | 0.1 | 0.1 | 0.2 | 0.2 | 0.2 |
| pH | 11.0 | 11.0 | 11.3 | 9.6 | 10.8 | 10.9 |
| Miscellaneous, sulfate and water | Up to 100% | | | | | |

**Example 18**

The following tablet detergent compositions were prepared according to the present invention by compression of a granular dishwashing detergent composition at a pressure of 13KN/cm$^2$ using a standard 12 head rotary press:

| | I | II | III | IV | V | VI | VII | VIII |
|---|---|---|---|---|---|---|---|---|
| STPP | - | 48.8 | 54.7 | 38.2 | - | 52.4 | 56.1 | 36.0 |
| Citrate | 20.0 | - | - | - | 35.9 | - | - | - |
| Carbonate | 20.0 | 5.0 | 14.0 | 15.4 | 8.0 | 23.0 | 20.0 | 28.0 |
| Silicate | 15.0 | 14.8 | 15.0 | 12.6 | 23.4 | 2.9 | 4.3 | 4.2 |
| Oxidoreductase of the present invention | 0.001 | 0.001 | 0.01 | 0.004 | 0.02 | 0.02 | 0.001 | 0.005 |
| Protease | 0.042 | 0.072 | 0.042 | 0.031 | 0.052 | 0.023 | 0.023 | 0.029 |
| Amylase | 0.012 | 0.012 | 0.012 | 0.007 | 0.015 | 0.003 | 0.017 | 0.002 |
| Lipase | 0.005 | - | - | - | - | - | - | - |
| PB1 | 14.3 | 7.8 | 11.7 | 12.2 | - | - | 6.7 | 8.5 |
| P B4 | - | - | - | - | 22.8 | - | 3.4 | - |
| Percarbonate | - | - | - | - | - | 10.4 | - | - |
| BB1 | 0.2 | - | 0.5 | - | 0.3 | 0.2 | - | - |
| BB2 | - | 0.2 | - | 0.5 | - | - | 0.1 | 0.2 |
| Nonionic | 1.5 | 2.0 | 2.0 | 2.2 | 1.0 | 4.2 | 4.0 | 6.5 |
| PAAC | - | - | 0.02 | 0.009 | - | - | - | - |
| MnTACN | - | - | - | - | 0.007 | - | - | - |
| TAED | 2.7 | 2.4 | - | - | - | 2.1 | 0.7 | 1.6 |
| HEDP | 1.0 | - | - | 0.9 | - | 0.4 | 0.2 | - |
| DETPMP | 0.7 | - | - | - | - | - | - | - |
| Paraffin | 0.4 | 0.5 | 0.5 | 0.5 | - | - | 0.5 | - |
| BTA | 0.2 | 0.3 | 0.3 | 0.3 | 0.3 | 0.3 | 0.3 | - |
| Polycarboxylate | 4.0 | - | - | - | 4.9 | 0.6 | 0.8 | - |
| PEG | - | - | - | - | - | 2.0 | - | 2.0 |
| Glycerol | - | - | - | - | - | 0.4 | - | 0.5 |
| Perfume | - | - | - | 0.05 | 0.2 | 0.2 | 0.2 | 0.2 |

(continued)

| | I | II | III | IV | V | VI | VII | VIII |
|---|---|---|---|---|---|---|---|---|
| Weight of tablet | 20g | 25g | 20g | 30g | 18g | 20g | 25g | 24g |
| pH | 10.7 | 10.6 | 10.7 | 10.7 | 10.9 | 11.2 | 11.0 | 10.8 |
| Miscellaneous, sulfate and water | Up to 100% | | | | | | | |

**Example 19**

The following compact high density (0.96Kg/l) dishwashing detergent compositions were prepared according to the present invention :

| | I | II | III | IV | V | VI |
|---|---|---|---|---|---|---|
| STPP | - | 51.0 | 51.0 | - | - | 44.3 |
| Citrate | 17.0 | - | - | 50.0 | 40.2 | - |
| Carbonate | 17.5 | 14.0 | 20.0 | - | 8.0 | 33.6 |
| Bicarbonate | - | - | - | 26.0 | - | - |
| Silicate | 15.0 | 15.0 | 8.0 | - | 25.0 | 3.6 |
| Metasilicate | 2.5 | 4.5 | 4.5 | - | - | - |
| PB1 | 10.0 | 8.0 | 8.0 | - | - | - |
| PB4 | - | - | - | 10.0 | - | - |
| Percarbonate | - | - | - | - | 11.8 | 4.8 |
| Nonionic | 2.0 | 1.5 | 1.5 | 3.0 | 1.9 | 5.9 |
| DAP 1 | 0.2 | 1.0 | 4.3 | 6.7 | 1.7 | 0.3 |
| TAED | 2.0 | - | - | 4.0 | - | 1.4 |
| HEDP | 1.0 | - | - | - | - | - |
| DETPMP | 0.6 | - | - | - | - | - |
| MnTACN | - | - | - | - | 0.01 | - |
| PAAC | - | 0.01 | 0.01 | - | - | - |
| Paraffin | 0.5 | 0.4 | 0.4 | 0.6 | - | - |
| Oxidoreductase of the present invention | 0.04 | 0.001 | 0.03 | 0.005 | 0.005 | 0.005 |
| Protease | 0.072 | 0.053 | 0.053 | 0.026 | 0.059 | 0.01 |
| Amylase | 0.012 | 0.012 | 0.012 | 0.021 | 0.021 | 0.006 |
| Lipase | - | 0.001 | - | 0.005 | - | - |
| BTA | 0.3 | 0.2 | 0.2 | 0.3 | 0.3 | 0.3 |
| Polycarboxylate | 6.0 | - | - | - | 4.0 | 0.9 |
| Perfume | 0.2 | 0.1 | 0.1 | 0.2 | 0.2 | 0.2 |
| pH | 11.0 | 11.0 | 11.3 | 9.6 | 10.8 | 10.9 |
| Miscellaneous, sulfate and water | Up to 100% | | | | | |

**Example 20**

The following tablet detergent compositions were prepared according to the present invention by compression of a granular dishwashing detergent composition at a pressure of $13KN/cm^2$ using a standard 12 head rotary press:

| | I | II | III | IV | V | VI | VII | VIII |
|---|---|---|---|---|---|---|---|---|
| STPP | - | 48.8 | 54.7 | 38.2 | - | 52.4 | 56.1 | 36.0 |
| Citrate | 20.0 | - | - | - | 35.9 | - | - | - |
| Carbonate | 20.0 | 5.0 | 14.0 | 15.4 | 8.0 | 23.0 | 20.0 | 28.0 |
| Silicate | 15.0 | 14.8 | 15.0 | 12.6 | 23.4 | 2.9 | 4.3 | 4.2 |
| Oxidoreductase of the present invention | 0.1 | 0.001 | 0.01 | 0.4 | 0.02 | 0.02 | 0.1 | 0.005 |
| Protease | 0.042 | 0.072 | 0.042 | 0.031 | 0.052 | 0.023 | 0.023 | 0.029 |

(continued)

| | I | II | III | IV | V | VI | VII | VIII |
|---|---|---|---|---|---|---|---|---|
| Amylase | 0.012 | 0.012 | 0.012 | 0.007 | 0.015 | 0.003 | 0.017 | 0.002 |
| Lipase | 0.005 | - | - | - | - | - | - | - |
| PB1 | 14.3 | 7.8 | 11.7 | 12.2 | - | - | 6.7 | 8.5 |
| PB4 | - | - | - | - | 22.8 | - | 3.4 | - |
| Percarbonate | - | - | - | - | - | 10.4 | - | - |
| DAP 1 | 0.6 | 0.8 | 1.0 | 1.2 | 1.1 | 0.8 | 0.5 | 1.4 |
| Nonionic | 1.5 | 2.0 | 2.0 | 2.2 | 1.0 | 4.2 | 4.0 | 6.5 |
| PAAC | - | - | 0.02 | 0.009 | - | - | - | - |
| MnTACN | - | - | - | - | 0.007 | - | - | - |
| TAED | 2.7 | 2.4 | - | - | - | 2.1 | 0.7 | 1.6 |
| HEDP | 1.0 | - | - | 0.9 | - | 0.4 | 0.2 | - |
| DETPMP | 0.7 | - | - | - | - | - | - | - |
| Paraffin | 0.4 | 0.5 | 0.5 | 0.5 | - | - | 0.5 | - |
| BTA | 0.2 | 0.3 | 0.3 | 0.3 | 0.3 | 0.3 | 0.3 | - |
| Polycarboxylate | 4.0 | - | - | - | 4.9 | 0.6 | 0.8 | - |
| PEG | - | - | - | - | - | 2.0 | - | 2.0 |
| Glycerol | - | - | - | - | - | 0.4 | - | 0.5 |
| Perfume | - | - | - | 0.05 | 0.2 | 0.2 | 0.2 | 0.2 |
| Weight of tablet | 20g | 25g | 20g | 30g | 18g | 20g | 25g | 24g |
| pH | 10.7 | 10.6 | 10.7 | 10.7 | 10.9 | 11.2 | 11.0 | 10.8 |
| Miscellaneous, sulfate and water | Up to 100% | | | | | | | |

**Example 21**

The following compact high density (0.96Kgll) dishwashing detergent compositions were prepared according to the present invention :

| | I | II | III | IV | V | VI |
|---|---|---|---|---|---|---|
| STPP | - | 51.0 | 51.0 | - | - | 44.3 |
| Citrate | 17.0 | - | - | 50.0 | 40.2 | - |
| Carbonate | 17.5 | 14.0 | 20.0 | - | 8.0 | 33.6 |
| Bicarbonate | - | - | - | 26.0 | - | - |
| Silicate | 15.0 | 15.0 | 8.0 | - | 25.0 | 3.6 |
| Metasilicate | 2.5 | 4.5 | 4.5 | - | - | - |
| Catalyst | 0.01 | 0.005 | 0.1 | 2.0 | 0.01 | 0.005 |
| PB1 | 10.0 | 8.0 | 8.0 | - | - | - |
| PB4 | - | - | - | 10.0 | - | - |
| Percarbonate | - | - | - | - | 11.8 | 4.8 |
| Nonionic | 2.0 | 1.5 | 1.5 | 3.0 | 1.9 | 5.9 |
| TAED | 2.0 | - | - | 4.0 | - | 1.4 |
| HEDP | 1.0 | - | - | - | - | - |
| DETPMP | 0.6 | - | - | - | - | - |
| PAAC | - | 0.01 | 0.01 | - | - | - |
| Paraffin | 0.5 | 0.4 | 0.4 | 0.6 | - | - |
| Oxidoreductase of the present invention | 0.04 | 0.001 | 0.03 | 0.005 | 0.005 | 0.005 |
| Protease | 0.072 | 0.053 | 0.053 | 0.026 | 0.059 | 0.01 |
| Amylase | 0.012 | 0.012 | 0.012 | 0.021 | 0.021 | 0.006 |
| Lipase | - | 0.001 | - | 0.005 | - | - |
| BTA | 0.3 | 0.2 | 0.2 | 0.3 | 0.3 | 0.3 |

(continued)

| | I | II | III | IV | V | VI |
|---|---|---|---|---|---|---|
| Polycarboxylate | 6.0 | - | - | - | 4.0 | 0.9 |
| Perfume | 0.2 | 0.1 | 0.1 | 0.2 | 0.2 | 0.2 |
| pH | 11.0 | 11.0 | 11.3 | 9.6 | 10.8 | 10.9 |
| Miscellaneous, sulfate and water | Up to 100% | | | | | |

**Example 22**

The following tablet detergent compositions were prepared according to the present invention by compression of a granular dishwashing detergent composition at a pressure of $13KN/cm^2$ using a standard 12 head rotary press:

| | I | II | III | IV | V | VI | VII | VIII |
|---|---|---|---|---|---|---|---|---|
| STPP | - | 48.8 | 54.7 | 38.2 | - | 52.4 | 56.1 | 36.0 |
| Citrate | 20.0 | - | - | - | 35.9 | - | - | - |
| Carbonate | 20.0 | 5.0 | 14.0 | 15.4 | 8.0 | 23.0 | 20.0 | 28.0 |
| Silicate | 15.0 | 14.8 | 15.0 | 12.6 | 23.4 | 2.9 | 4.3 | 4.2 |
| Oxidoreductase of the present invention | 0.001 | 0.001 | 0.01 | 0.004 | 0.02 | 0.02 | 0.001 | 0.005 |
| Protease | 0.042 | 0.072 | 0.042 | 0.031 | 0.052 | 0.023 | 0.023 | 0.029 |
| Amylase | 0.012 | 0.012 | 0.012 | 0.007 | 0.015 | 0.003 | 0.017 | 0.002 |
| Lipase | 0.005 | - | - | - | - | - | - | - |
| Catalyst | 0.001 | 0.003 | 0.05 | 0.001 | 0.001 | 0.003 | 0.01 | 0.001 |
| PB1 | 14.3 | 7.8 | 11.7 | 12.2 | - | - | 6.7 | 8.5 |
| PB4 | - | - | - | - | 22.8 | - | 3.4 | - |
| Percarbonate | - | - | - | - | - | 10.4 | - | - |
| Nonionic | 1.5 | 2.0 | 2.0 | 2.2 | 1.0 | 4.2 | 4.0 | 6.5 |
| PAAC | - | - | 0.02 | 0.009 | - | - | - | - |
| TAED | 2.7 | 2.4 | - | - | - | 2.1 | 0.7 | 1.6 |
| HEDP | 1.0 | - | - | 0.9 | - | 0.4 | 0.2 | - |
| DETPMP | 0.7 | - | - | - | - | - | - | - |
| Paraffin | 0.4 | 0.5 | 0.5 | 0.5 | - | - | 0.5 | - |
| BTA | 0.2 | 0.3 | 0.3 | 0.3 | 0.3 | 0.3 | 0.3 | - |
| Polycarboxylate | 4.0 | - | - | - | 4.9 | 0.6 | 0.8 | - |
| PEG | - | - | - | - | - | 2.0 | - | 2.0 |
| Glycerol | - | - | - | - | - | 0.4 | - | 0.5 |
| Perfume | - | - | - | 0.05 | 0.2 | 0.2 | 0.2 | 0.2 |
| Weight of tablet | 20g | 25g | 20g | 30g | 18g | 20g | 25g | 24g |
| pH | 10.7 | 10.6 | 10.7 | 10.7 | 10.9 | 11.2 | 11.0 | 10.8 |
| Miscellaneous, sulfate and water | Up to 100% | | | | | | | |

**Example 23**

The following liquid rinse aid compositions were prepared according to the present invention :

| | I | II | III | IV |
|---|---|---|---|---|
| Oxidoreductase of the present invention | 0.001 | 0.0005 | 0.01 | 0.001 |
| Catalyst | 0.1 | 0.01 | 0.008 | 0.001 |
| Nonionic | 10.0 | 13.6 | 62.3 | 60.0 |
| Propylene glycol | - | - | 5.0 | 5.5 |
| Citric | 3.5 | 4.6 | - | - |
| SCS | 10.0 | 7.7 | - | - |

(continued)

| | I | II | III | IV |
|---|---|---|---|---|
| pH of the liquid | 3.0 | 2.5 | 7.2 | 7.2 |
| Miscellaneous, solvent and water | Up to 100% | | | |

**Example 24**

The following automatic dishwashing tablets were made in accordance with the present invention (g of raw material and enzymes are expressed in pure enzyme) :

| | I | II | III | IV | V | VI |
|---|---|---|---|---|---|---|
| Phase 1 | | | | | | |
| STPP | 9.6 | 9.6 | 10.6 | 9.6 | 9.6 | 10.6 |
| Silicate | 0.5 | 0.7 | 2.9 | 0.5 | 0.7 | 2.9 |
| SKS-6 | 1.5 | 1.5 | - | 1.5 | 1.5 | - |
| Carbonate | 2.3 | 2.7 | 2.8 | 2.3 | 2.7 | 2.8 |
| HEDP | 0.2 | 0.2 | 0.2 | 0.2 | 0.2 | 0.2 |
| PB1 | 2.4 | 2.4 | 2.8 | 2.4 | 2.4 | 2.8 |
| PAAC | 0.002 | 0.002 | - | - | - | - |
| Catalyst | - | - | - | 0.002 | 0.002 | - |
| BB1 | 0.2 | 0.5 | - | - | - | - |
| DAP 1 | - | - | 0.5 | - | - | 0.2 |
| Amylase | 0.1 | 0.1 | 0.001 | 0.1 | 0.1 | 0.001 |
| Protease | 0.06 | 0.06 | 0.002 | 0.06 | 0.06 | 0.002 |
| Nonionic | 0.4 | 0.8 | 0.4 | 0.4 | 0.8 | 0.4 |
| PEG 6000 | 0.4 | 0.26 | - | 0.4 | 0.26 | - |
| BTA | 0.04 | 0.04 | 0.06 | 0.04 | 0.04 | 0.06 |
| Paraffin | 0.1 | 0.10 | 0.1 | 0.1 | 0.10 | 0.1 |
| Perfume | 0.02 | 0.02 | 0.02 | 0.02 | 0.02 | 0.02 |
| Total | 17.7g | 18.5g | 20.1g | 17.7g | 18.5g | 20.1g |
| Phase 2 | | | | | | |
| Oxidoreductase of the present invention | 0.005 | 0.5 | 0.2 | 0.005 | 0.5 | 0.2 |
| Amylase | 0.003 | 0.003 | 0.004 | 0.003 | 0.003 | 0.004 |
| Protease | 0.01 | 0.009 | 0.01 | 0.01 | 0.009 | 0.01 |
| Citric acid | 0.3 | - | 0.6 | 0.3 | - | 0.6 |
| Sulphamic acid | - | 0.3 | - | - | 0.3 | - |
| Bicarbonate | 1.1 | 0.4 | 0.6 | 1.1 | 0.4 | 0.6 |
| Carbonate | - | 0.5 | - | - | 0.5 | - |
| Triacetin | - | - | 1.2 | - | - | 1.2 |
| $CaCl_2$ | - | 0.07 | 0.1 | - | 0.07 | 0.1 |
| PEG 6000 | - | - | 1.2 | - | - | 1.2 |
| PEG 3000 | 0.06 | 0.06 | - | 0.06 | 0.06 | - |
| Total | 2.05g | 2.50g | 23.6g | 2.05g | 2.50g | 23.6g |

The tablet compositions I and II are prepared as follows. The detergent active composition of phase 1 is prepared by admixing the granular and liquid components and is then passed into the die of a conventional rotary press. The press includes a punch suitably shaped for forming a mould. The cross-section of the die is approximately 30x38 mm. The composition is then subjected to a compression force of 940 kg/$cm^2$ and the punch is then elevated exposing the first phase of the tablet containing the mould in its upper surface. The detergent active composition of phase 2 is prepared in similar manner and is passed into the die. The particulate active composition is then subjected to a compression force of 170 kg/$cm^2$, the punch is elevated, and the multi-phase tablet ejected from the tablet press.

The resulting tablets dissolve or disintegrate in a washing machine as described above within 12 minutes, phase 2 of the tablets dissolving within 5 minutes. The tablets display improved strength, especially on long-term storage, together with excellent dissolution characteristics.

The tablet composition III was prepared as follows : The compressed portion is prepared by delivering the composition of active detergent components to a punch cavity of a modified rotary tablet press and compressing the composition at a pressure of 940kg/cm$^2$. The modified tablet press provides tablet wherein the compressed portion has a mould. For the purposes of Example III, the non-compressed portion is in particulate form. The non-compressed portion is accurately delivered to the mould of the compressed portion using a nozzle feeder. The non-compressed portion is adhered to the compressed portion by coating the non-compressed portion with a coating layer which contacts the compressed portion.

## Claims

**1.** An isolated nucleic acid encoding an open reading frame for a carotene-degrading oxidoreductase, comprising

(a) a sequence according to SEQ ID NO: 1, or
(b) a sequence having 75%, preferably 80%, more preferably 90%, more preferably 95% or more sequence identity with the sequence according to (a), or
(c) a sequence capable of hybridising to the sequence of (a) and/or (b) under stringent conditions, and/or
(d) a sequence that is complementary to (a), (b) and/or (c).

**2.** The nucleic acid according to claim 1, wherein the sequence of the nucleic acid is derived from fungus or yeast, preferably a basidiomycete.

**3.** The nucleic acid according to claim 2, wherein the sequence of the nucleic acid is derived from *Lepista irina.*

**4.** A vector comprising the sequence of a nucleic acid according to any one of claims 1 to 3.

**5.** A cell transformed with the nucleic acid according to any one of claims 1 to 3 or with the vector according to claim 4.

**6.** A cell culture comprising cells according to claim 5 and a suitable cell culture medium.

**7.** A polypeptide encoded by the nucleic acid according to SEQ ID NO: 1.

**8.** The polypeptide according to claim 7 having

(a) an amino acid sequence according to SEQ ID NO: 2,
(b) an amino acid sequence with 70%, preferably 80%, more preferably 90% homology or more with (a), and/or
(c) an amino acid sequence which is immunologically cross-reactive with (a) and/or (b).

**9.** The polypeptide according to claim 7 or 8, being active in the conversion of a carotenoid substrate.

**10.** The polypeptide according to claim 9, having a substrate specificity for β,β-carotene, α-carotene, lycopene, capsanthin, lutein, antheraxanthin, violaxanthin, zeaxanthin, astaxanthin, canthaxanthin, luteoxanthin, neoxanthin, and the respective apo-carotenoids.

**11.** An oxidoreductase active in converting carotenoid substrates isolated from yeast or fungus, having a molecular weight of about 50 kDa and an iso-electric point of about 3.75.

**12.** The carotene-degrading oxidoreductase of claim 11, wherein it cleaves carotenoids asymmetrically.

**13.** The carotene-degrading oxidoreductase of claim 11 or 12, wherein it is derived from *Lepista irina.*

**14.** A detergent composition, comprising a microbial oxidoreductase capable of converting carotenoid substrates.

**15.** The detergent composition according to claim 14, wherein the microbial oxidoreductase is the oxidoreductase according to any one of claims 11 to 13 or the polypeptide according to any one of claims 7 to 10.

**16.** The detergent composition according to claim 14 or 15, further comprising a surfactant, dispersant, balance carrier and/or adjunct ingredient.

**17.** The detergent composition according to any one of claims 14 to 16, further comprising an additional enzyme or enzyme system.

**18.** The detergent composition according to claim 17, wherein the additional enzyme is a carotene-specific esterase.

**19.** The detergent composition according to any one of claims 14 to 18, substantially free of hydrogen peroxide.

**20.** A method for treating carotene-comprising stains, comprising contacting the material bearing the stain with the polypeptide or oxidoreductase according to any one of claims 7 to 13, or the detergent composition according to any one of claims 14 to 19.

**21.** A method for producing carotene-derived products from a carotenoid substrate, comprising:

(a) contacting the carotenoid precursor with the polypeptide or oxidoreductase according to any one of claims 7 to 12, or with the detergent composition according to any one of claims 13 to 17, and
(b) incubating the mixture of carotenoid precursor and oxidoreductase.

**22.** The method according to claim 20, wherein the carotene-derived products are isolated and/or purified.

**23.** The method according to claim 20 or 21, wherein it is carried out in the absence of hydrogen peroxide.

**24.** Use of the oxidoreductase of any of claims 11 to 13 or the polypeptide of any of claims 7 to 10 in the treatment of stains.

**25.** Use of the oxidoreductase of any of claims 11 to 13 or the polypeptide of any of claims 7 to 10 in the conversion of carotenoid substrates.

**Figure 1**

cDNA sequence

GGATCCGGCCATTATGGCCGGGGAAGATCCCCAGTCTTACAACCCACTGC
A**ATG**TCTTTCAAGACGCTCTCCGCTCTCGCGCTTGCGCTCGGCGCCGCCG
TCCAGTTCGCGAGTGCTGCTGTGCCTCTCGTCCAGAAACGCGCAACTTGC
GCCGACGGACGCACCACCGCAAATGCTGCATGTTGCGTTCTGTTCCCCAT
CCTCGATGACATCCAAGAAAACCTCTTCGACGGTGCCCAGTGTGGAGAAG
AGGTACACGAGTCCCTTCGTTTGACTTTCCACGATGCAATCGGTTTCTCTC
CTACTTTAGGCGGAGGAGGAGCTGACGGTTCCATCATCGCGTTCGACACC
ATTGAGACTAATTTCCCCGCCAATGCTGGCATCGATGAAATCGTCAGCGCT
CAGAAGCCATTCGTGGCTAAACACAACATCTCCGCCGGCGACTTCATTCAA
TTTGCTGGCGCCGTTGGAGTCTCCAACTGCCCTGGTGGTGTCAGGATTCC
TTTCTTCTTGGGTCGCCCGGATGCCGTGGCGGCCTCCCCGGACCACCTC
GTGCCAGAGCCTTTTGATTCTGTTGACACCATTCTTGCCAGAATGGGTGAC
GCAGGCTTCAGTCCCGTCGAGGTTGTTTGGCTCCTGGCTTCGCACTCCAT
TGCCGCTGCCGACAAGGTTGACCCATCGATTCCTGGAACGCCATTCGATT
CAACCCCCGGAGTTTTTGATTCTCAATTCTTCATCGAAACGCAACTTAAAG
GCAAACTCTTCCCAGGCACTGCTGACAACAAGGGAGAAGCCCAATCTCCA
TTGCAAGGAGAGATCAGGCTTCAGTCCGATCACTTGTTGGCTAGAGACCC
CCAGACTGCCTGTGAATGGCAGTCCATGGTTAACAACCAACCGAAGATTC
AGAACCGTTTCGCTGCTACCATGTCGAAGATGGCTCTTCTTGGCCAAGACA
AGACCAAATTGATTGACTGTTCTGATGTTATCCCCACCCCTCCTGCCCTTG
TCGGAGCGGCCCACTTGCCGGCGGGATTTTCTCTTAGCGATGTAGAGCAA
GCGTGCGCCGAGACCCCTTTCCCTGCTCTTACTGCTGACCCAGGCCCAGT
AACCTCTGTCCCTCCCGTCCCT**GGATCGTAA**ATGCTTCGATACCTGAATAT
GCTCGTTCTGCTGCGCTGAATTTCCAACTTTTGCCATTGGGTCTGTATTCG
ATTCTAGATGTTTGTGATATCAACTGTGTATAAATGATCTTTTGAAATATACT
TTTTTCTGCGGAGPolyA

***Figure 2***

Protein sequence

MSFKTLSALALALGAAVQFASAAVPLVQKRATCADGRTTANAACCVLFPILDDI
QENLFDGAQCGEEVHESLRLTFHDAIGFSPTLGGGGADGSIIAFDTIETNFPAN
AGIDEIVSAQKPFVAKHNISAGDFIQFAGAVGVSNCPGGVRIPFFLGRPDAVAA
SPDHLVPEPFDSVDTILARMGDAGFSPVEVVWLLASHSIAAADKVDPSIPGTPF
DSTPGVFDSQFFIETQLKGKLFPGTADNKGEAQSPLQGEIRLQSDHLLARDPQ
TACEWQSMVNNQPKIQNRFAATMSKMALLGQDKTKLIDCSDVIPTPPALVGAA
HLPAGFSLSDVEQACAETPFPALTADPGPVTSVPPVPG

Figure 3

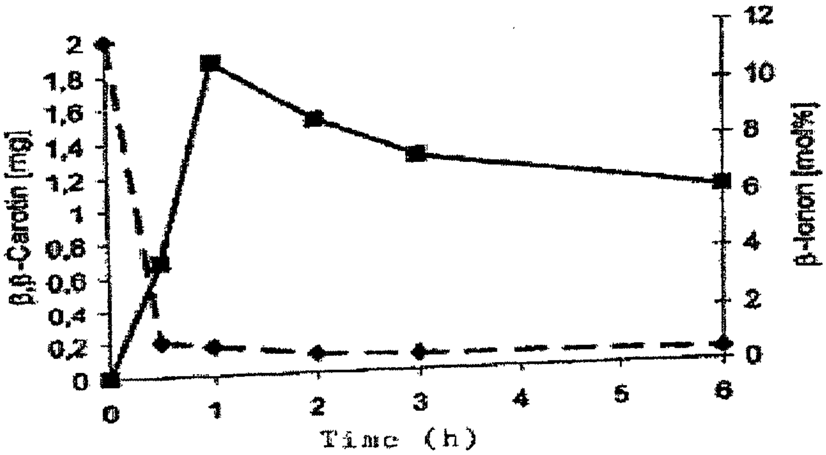


Figure 4

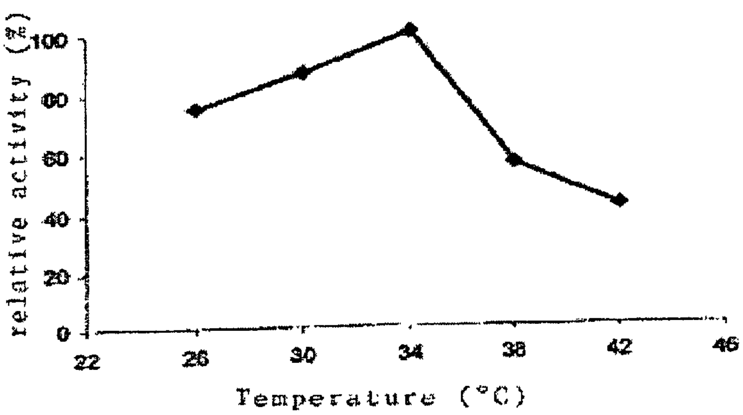


Figure 5

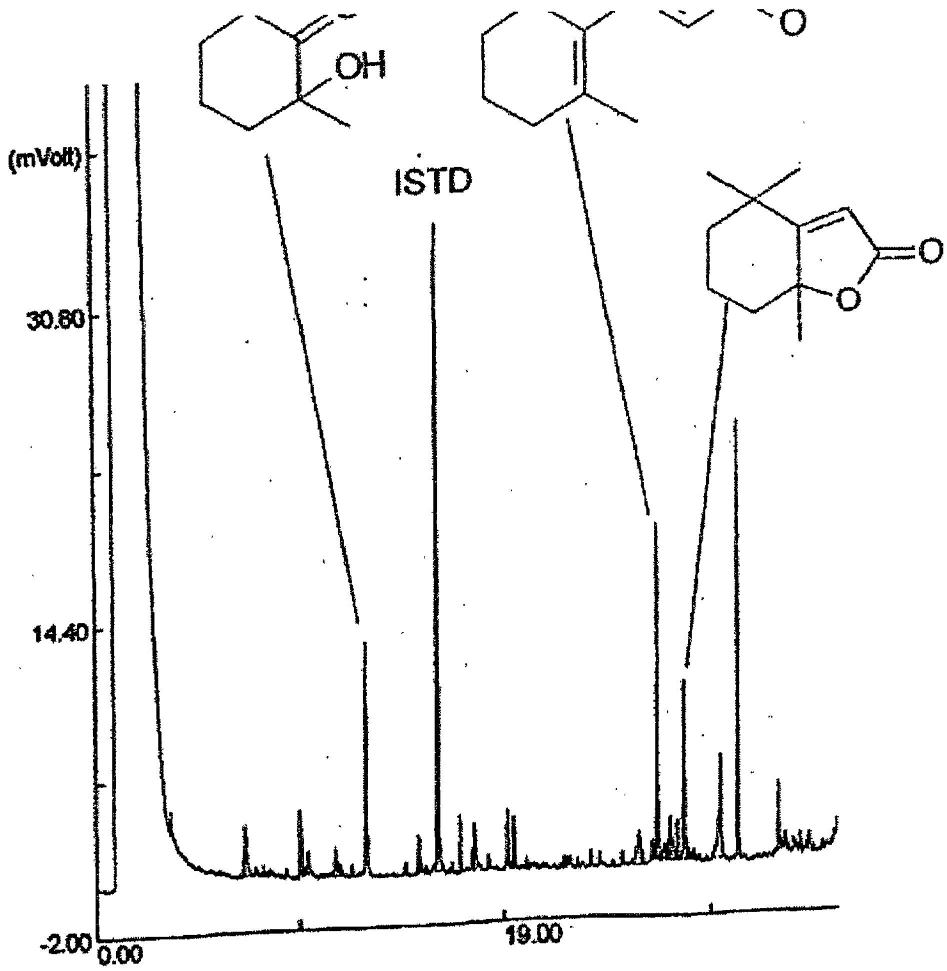
(mVolt)
30.80
14.40
-2.00
0.00
19.00
ISTD
OH

Figure 6

(A)

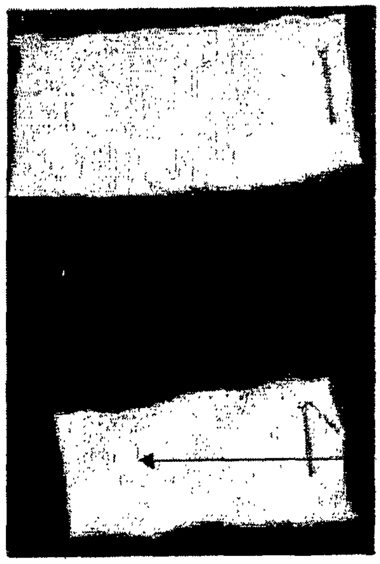

(B)

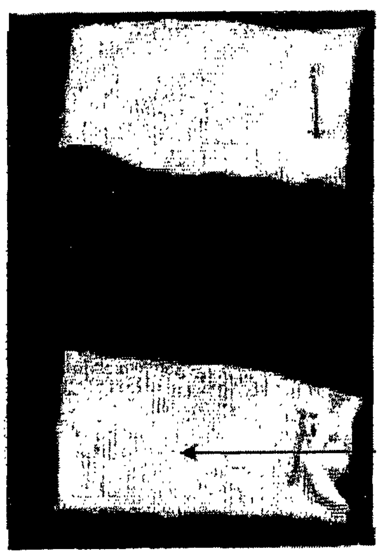

European Patent Office

# EUROPEAN SEARCH REPORT

Application Number
EP 02 44 7168

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | RUIZ-DUENAS FRANCISCO JAVIER ET AL: "Molecular characterization of a novel peroxidase isolated from the ligninolytic fungus Pleurotus eryngii." MOLECULAR MICROBIOLOGY, vol. 31, no. 1, January 1999 (1999-01), pages 223-235, XP008013345 ISSN: 0950-382X | 1-6,8-12 | C12N15/53<br>C12N9/02<br>C11D3/386<br>C12N1/21<br>C12N5/10 |
| Y | * page 223 - page 227; figure 3 * | 7,15,19, 20,24,25 | |
| X | WO 01 92454 A (UNILEVER PLC ;LEVER HINDUSTAN LTD (IN); UNILEVER NV (NL)) 6 December 2001 (2001-12-06) | 14,16,17 | |
| Y | * page 2 - page 4 * | 7,15,19, 20,24,25 | |
| | * page 9 - page 17 *<br>* page 19 * | | |
| A | MOKEEVA V L ET AL: "OXIDASES IN THE FRUIT BODIES OF SOME BASIDIOMYCETES" MIKOLOGIYA I FITOPATOLOGIYA, vol. 22, no. 3, 1988, pages 235-239, XP008013412 ISSN: 0026-3648<br>* page 237 * | | TECHNICAL FIELDS SEARCHED (Int.Cl.7)<br>C12N<br>C11D |
| A | GELINAS P ET AL: "Oxido-reductases and lipases as dough-bleaching agents." CEREAL CHEMISTRY, vol. 75, no. 6, November 1998 (1998-11), pages 810-814, XP002230174 ISSN: 0009-0352<br>* the whole document * | | |
| | -/-- | | |

The present search report has been drawn up for all claims

| Place of search | Date of completion of the search | Examiner |
|---|---|---|
| THE HAGUE | 13 February 2003 | Schneider, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 02 44 7168

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int.Cl.7) |
| A | HOFRICHTER MARTIN: "Review: Lignin conversion by manganese peroxidase (MnP)." ENZYME AND MICROBIAL TECHNOLOGY, vol. 30, no. 4, 2002, pages 454-466, XP002230175 16 April, 2002 ISSN: 0141-0229 * the whole document * ----- | | |
| | | | TECHNICAL FIELDS SEARCHED (Int.Cl.7) |

The present search report has been drawn up for all claims

| Place of search | Date of completion of the search | Examiner |
|---|---|---|
| THE HAGUE | 13 February 2003 | Schneider, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 02 44 7168

This annex lists the patent family membersrelating to the patent documents cited in the above-mentioned European search report.
The members are as contained in the European Patent Office EDP file on
The European Patent Office is in no way liable for these particulars which are merely given for the purpose of information.

13-02-2003

| Patent document cited in search report | Publication date | Patent family member(s) | Publication date |
|---|---|---|---|
| WO 0192454 A | 06-12-2001 | AU 6229101 A | 11-12-2001 |
| | | WO 0192454 A1 | 06-12-2001 |
| | | US 2002016279 A1 | 07-02-2002 |
| | | AU 2173602 A | 15-05-2002 |
| | | WO 0236724 A1 | 10-05-2002 |
| | | US 2002119900 A1 | 29-08-2002 |

EPO FORM P0459

For more details about this annex : see Official Journal of the European Patent Office, No. 12/82